# EUROPEAN PATENT APPLICATION

(11) **EP 2 371 421 A1**
(43) Date of publication of application: **05.10.2011**
(21) Application number: 10155495.4
(22) Date of filing: 04.03.2010
(51) Int. Cl.: A61P 25/28, A61K 31/4178, A61K 31/4164, A61K 31/404, C07D 233/64, C07D 401/12, C07D 403/12, C07D 413/06, C07D 209/20, C07K 5/02, C07K 5/06

(54) **Ligands of insulin degrading enzyme and their uses**

(71) Applicant: Université de Lille 2 Droit et Santé, 59800 Lille (FR)
(72) Inventor: DEPREZ-POULAIN, Rebecca Franziska, 59000, LILLE (FR); CHARTON, Julie Jeanne Marine, 59320, HAUBOURDIN (FR); DEPREZ, Benoit Gaston Pierre, 59000, LILLE (FR); LEROUX, Florence Catherine Gilberte, 59175, TEMPLEMARS (FR); GAURIOT, Marion, 59000, LILLE (FR); TANG, Wei-Jen, CHICAGO, IL 60611 (US); TOTOBENAZARA, Jane, 59800, LILLE (FR)
(74) Representative: Noel, Chantal Odile

(57) **Abstract**

The invention relates to ligands of insulin degrading enzyme and to their uses.
The ligands of the invention are non peptidic and bind specifically to the exosite of insulin degrading enzyme.
They are useful, in particular a use in the pharmaceutical field.

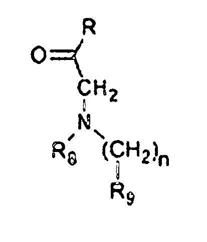

## Description

The present invention relates to compounds which are ligands of the exosite of insulin degrading enzyme (IDE) and to their uses as modulators of insulin degrading enzyme activity or in a method for screening modulators of IDE activity.

It also relates to a pharmaceutical composition comprising these compounds.

Insulin degrading enzyme (insulysin, insulinase, IDE) is a highly conserved 110 kDa zinc metalloprotease (M16 family) that is involved in the clearance of various physiologically relevant peptide substrates (1, 2, 3, 4, 19, 20, 21).

It was first discovered based on its high affinity to bind insulin and degrade it (5) and named on the basis of this high affinity for insulin. As insulin is a hormone that plays a central role in glucose homeostasis and the development of diabetes in human, IDE was suspected to be involved in the physiopathology of diabetes.

In addition to insulin degradation, IDE has also been implicated in the degradation of the amyloid-β peptide (Aβ) *in vitro* (6). Consistent with the *in vitro* degradation of insulin and Aβ by IDE, mice with the knock-out of the IDE gene and the GK rat which has known missense mutations in the IDE gene display elevated insulin levels, glucose intolerance and elevated levels of Aβ in the brain (7, 8, 9). In addition, a nucleotide polymorphism of the human IDE gene is linked to type 2 diabetes and a substantial body of genetic evidence implicates variations in and around the *Ide* gene with the incidence and onset of Alzheimer Disease (AD) (13, 14, 15).

IDE cleaves also several other pharmacologically potent peptides such as Insulin-Growth Factor II (IGF-II), atrial natriuretic peptide (ANP), transforming growth factor α (TGF-α), amylin, glucagon, bradykinin, kallidin, beta-endorphin and somatostatin (19, 20, 21).

Paradoxically, even though IDE has a broad range of substrates, it exhibits a remarkable capacity to selectively cleave certain hormones without degrading related family members (23).

Interestingly, while IDE peptide substrates share little or no homology of primary amino acid sequence, they are amyloidogenic in nature (16). The structural basis for substrate recognition and regulation of IDE can be understood based on crystal structures of substrate-bound and substrate-free human IDE (17, 18).

IDE is organized in two 56-KDa catalytic N- and C-terminal domains which are joined by an extended 28 amino acid residues loop, and enclose a large catalytic chamber that can fit peptides smaller than 70-amino acids long. IDE contains at its catalytic site a tetrad of conserved residues (His-Xaa-Xaa-Glu-His) that are involved in zinc binding and peptide hydrolysis (25).

Size and charge distribution of this catalytic chamber are properties likely involved in the regulation of recognition and binding of substrates. IDE also undergoes a conformational switch from a closed to an open state to allow entrance of the substrates in its catalytic chamber.

Tight interactions with substrates occur at an exosite located ∼ 30 Å away from the catalytic center that anchors the N-terminus of substrates to facilitate binding and subsequent multiple cleavage at the catalytic site of substrates by IDE via stochastic process (19).

Malito et al. (19) have shown that IDE also degrades peptide substrates that are too short to occupy both the catalytic site and the exosite simultaneously; indeed, they have studied the bradykinin-bound IDE structure and observed the binding of bradykinin at the exosite, not at the catalytic site.

The unique size, electrostatic potential and exosite of IDE's catalytic chamber are postulated as key factors for the selective binding and unfolding of IDE substrates (19, 21, 22).

The subcellular localization of IDE may differ depending on the specific cell type, where it can be found in the cytosol, on the cell surface, in endosomes, or as part of the extracellular milieu (23). The catalytic activity of IDE has been reported to be regulated by oligomerization. Moreover, the catalytic activity of IDE can be regulated by physiologically relevant ATP concentrations, long chain fatty acids, oxidative stress, and endogenous peptide inhibitors such as ubiquitin (23, 24, 26).

For instance, ATP inhibits IDE-mediated insulin degradation at physiological concentrations. ATP and related compounds were later found to dramatically activate the hydrolysis of shorter substrates by as much as ∼ 70 fold (23).

Im H et al. (23) focused on the importance of the closed conformation for regulating the activity of IDE and provide new molecular details that will facilitate the development of activators and inhibitors of IDE.

Some inhibitors of IDE have already been reported but present some drawbacks.
- They may be non-selective:
   * 1,10-phenantroline which acts by zinc chelation or
   * several known peptidic hydroxamic acids, binding to the catalytic site of IDE and inhibiting both IDE and canonical zinc-metalloproteases, these latter several orders of magnitude more potently than IDE;
   * bacitracin, which acts by steric blockage;
- They may be cytotoxic: thiol blocking agents such as p-hydroxy-mercuribenzoate and N-ethylmaleimide
- They may be biologically active: insulin.

Considering that hydroxamic acids are among the most potent zinc-metalloprotease inhibitors but may not be selective and are therefore regarded as poor drug candidates, Leissring et al., in WO 2008/156701, have described new peptidic hydroxamates, IDE-selective, based on the peptide FRWE or the peptide Xaa-RY; for zinc-binding; a hydroxamic acid moiety was added to the N-terminus (see Table 4 of WO 2008/156701). These peptidic inhibitors are small peptides containing 4 or 5 amino acids and binding only to the catalytic site by the zinc-binding hydroxamic acid moiety (i.e. not binding the exosite).

Furthermore Leissring et al. (WO 2008/156701) have shown, as it emerges from Table 2 of WO 2008/156701, that non peptidic hydroxamates that are in some cases potent and broad-spectrum zinc-metalloproteases inhibitors, lack efficacy in inhibiting IDE.

Activators of IDE have also been reported in EP 1 674 580: suramine which contains a naphthalene group is an activator of IDE activity and may be used for treating Alzheimer's disease; however suramine is poorly biovailable and is thus administrated by iv route.

However, considering the very complex activity of IDE, there is a need of modulators of IDE activity to be able to provide efficient and selective drugs either, activators or inhibitors of IDE, depending on the substrate of IDE to be stimulated or inactivated. Moreover, there is also a need for substrate specific modulators.

The Inventors have now found, surprisingly, that compounds of formula I below, which are non peptidic, bind specifically to the exosite of IDE, even in cases where R₉ is an hydroxamic acid and thus are IDE-selective ligands and modulators.

### Definitions

. Ligands of the exosite of IDE: the binding of the compounds of formula I to the exosite of IDE is evidenced by X-rays studies, after cocrystallisation of several compound of Formula I and IDE (IDE-CF- E111Q mutant) (21)) and X-rays diffraction by hanging drop vapor diffusion.
. Ligands of the exosite of IDE may be modulators, inhibitors or activators of the IDE activity, depending on the substrate. By binding only the exosite, they play a regulatory role in substrate binding and subsequent cleavage by IDE. For instance, they can modify the size, shape and physico-chemical properties of the catalytic chamber of IDE and this may enhance the substrate binding and cleavage by reducing the entropy of substrates corresponding, to short peptides; alternatively, they could reduce the cleavage by interfering with substrate binding.
. Activator of IDE: compound of Formula I that binds the exosite of IDE and increases the IDE activity.
. Inhibitor of IDE: compound of Formula I that binds the exosite of IDE and decreases or stops the IDE activity
. Modulator of IDE: compound of Formula I that binds the exosite of IDE and increases or decreases the IDE activity, depending on the substrate.
. Assay for identifying an activator or an inhibitor of IDE activity: the general method is described in the Patent Application EP 1 674 580 and is adapted as follows in the present Application: the substrate used for identifying activators and/or inhibitors of IDE activity may be any fluoregenic substrate of IDE and preferably short substrates such a fluoregenic peptide substrate V (7-methoxycoumarin-4-yl-acetyl-NPPGFSAFK-2,4-dinitrophenyl) or any flurorogenic peptide substrate such as those described in EP 1 674 580, of formula F-linker-P-Q, wherein F represents a fluorophore, P represents an IDE substrate and Q a quenching molecule; preferably, F corresponds to the fluorophore ATTO-655 (Sigma-Aldrich Fluka.Inc) attached to the N-terminus of said substrate through a linker (using ATTO-655 maleimide) via a Cys; P is preferably the amyloid-β fragment 16-32: KLVFFAED and Q is a W residue, able to quench (to decrease) the intensity of the fluorescence emission of the fluorophore. The principle of the method is therefore the following: IDE is contacted with the labelled substrate and a compound of Formula I. If the compound of Formula I is an activator, the amount of cleaved substrate will increase and will be detectable by an increase of fluorescence. If the compound of Formula I is an inhibitor, the amount of cleaved substrate will decrease and will be detectable by a decrease of fluorescence. If the compound of Formula I is neither an activator nor an inhibitor, there will be no change of fluorescence.

Alternatively, the activation or inhibition of IDE activity may be identified by HPLC. Briefly, proteolysis of the substrate is evaluated either by detecting the degradation products or by quantifying the residual substrate.

The compounds of the invention have the following formula I: 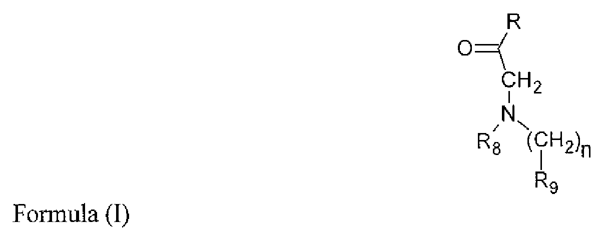 wherein:
**R**₈ is chosen among:
   . H,
   . a linear or branched C₁-C₇ alkyl group, or
   . a (linear or branched C₁-C₆ allcyl)-Ar group in which Ar is chosen in the group consisting of a phenyl, naphtyl, quinolein, indole, benzimidazole, benzothiazole, pyridine, pyrimidine, pyrazine group, optionally substituted with an halogen atom such as Cl, Br, I, F, a -CF₃ group, a linear or branched C₁-C₆ alkyl group, or a -O-(C₁-C₆ alkyl) group,
   . a (linear or branched C₁-C₅ alkyl)-O-Ar group in which Ar is chosen in the group consisting of a phenyl, naphtyl, quinolein, indole, benzimidazole, benzothiazole, pyridine, pyrimidine, pyrazine group, optionally substituted with an halogen atom such as Cl, Br, I, F, a -CF₃ group, a linear or branched C₁-C₆ alkyl group, or a -O-(C₁-C₆ alkyl) group,
**R**₉ is chosen in the group consisting of
   . a -COR' group in which R' is chosen among -OH, -O-(linear or branched C₁-C₄ alkyl) or -NHOH,
   . an isoster of a -COOH group, preferably a tetrazole, 1,2,4-oxadiazole-5-one group, a 1,2,4-oxadiazole-5-thione,
   . an hydroxamic acid group (-CONHOH),
   . a squaric acid group,
with the proviso that when R₉ is a squaric group, then n = 0, and when R₉ is different from a squaric acid group, then n = 1 or 2, and

**R** is a (L) or (D) aminoacid derivative having the following formula (II): 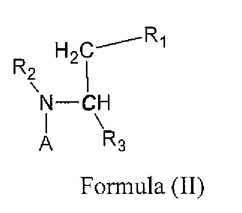 wherein:
- ***C*** designates a chiral carbon atom,
- **A** indicates the point of bonding of R to the corresponding carbon atom of the compound of formula I,
- **R₁** is chosen in the group consisting of
   . a 4-imidazole group which is non substituted, or N-substituted, preferably in tau position, by a methyl, benzyl or trityl group,
   . a S-linear or branched C₁-C₇ alkyl group, preferably a phenyl group, and optionally substituted by a C₅-C₁₀ (hetero)aromatic group, preferably a S-trityl group,
   . a ―CH₂-CH₂-guanidine group,
- **R**₂ is H or CH3, and
- **R**₃ is chosen in the group consisting of
   . a -COOR₄ group in which R₄ is H or a linear or branched C₁-C₇ alkyl group, or a C₁-C₇ cycloalkyl group,
   . a ―CONR₅R₆ group in which R₅ and R₆ are identical or different and are independently chosen in the group consisting of H, a C₁-C₇ alkyl group, a C₁-C₇ cycloalkyl group, and a benzyl group,
   . a -CH₂OH group, or
   . a group of the following formula: 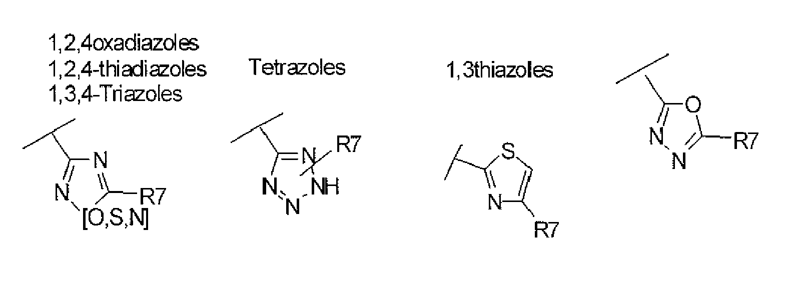
in which R₇ is a linear or branched C₁-C₇ alkyl group, and their pharmaceutically acceptable salts.

The compounds of the invention encompass the pharmaceutically acceptable salts of the compounds of formula **I**.

These salts are salts of the acid, *i.e.* of R₉ or salts of the nitrogen atoms, in formula **I**.

These salts are well known in the art and examples are given in "The Practice of Medicinal Chemistry" - Editor: Camille Georges Vermuth - Published in July 2008 ― Publisher : Elsevier Science and Technology Books.

The salts of the acid can be, in particular, sodium or potassium salts, or salts of organic acids, such as meglumin salts.

The salts of nitrogen may be hydrochloride, sulfate, formiate, maleate, fumarate, succinate or trifluoroacetate salts, in particular.

Preferred compounds are hereafter presented in subsets according to the preferred group R₉ (Formula I-1) or R₈ (Formulae I-2,I-3 and I-5) or R (Formulae I-4 and I-6), some of these preferred compounds being included in the definition of several subsets (compound No 24 for instance).

Preferred compounds of formula I are those in which R₉ is COR' with R' being chosen among -OH, -O-(linear or branched alkyl) or ―NHOH.

More preferably, R₉ is COOH or COOMe.

These compounds have the following formula I-1 : 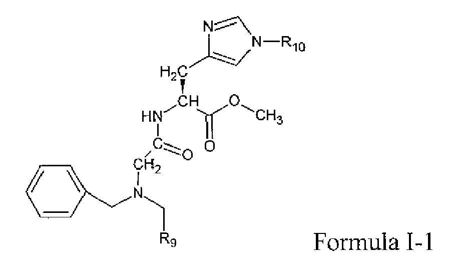 in which:
- R₉ and R₁₀ are chosen among the following groups:

| **R**₉ | **R**₁₀ |
|---|---|
| 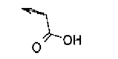 | H |
| 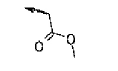 | H |
| H | H |
| 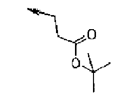 | Trityl |
| 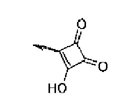 | H |
| 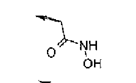 | H |
| 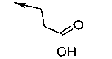 | H |

Indeed, compounds of formula I in which R represents a histidyl derivative of formula II in which R₁ has the following formula: 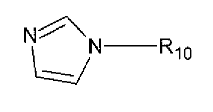 R₈ represents ―(CH₂)-phenyl
and R₉ is chosen among a -COOH group, a squaric group and a hydroxamic group are inhibitors of IDE activities measured according to the assay specified in the definitions.

The *in vitro* inhibition activities on IDE of these compounds are reported in the following Table 1.

**Table 1**

| **R**₉ | **R**₁₀ | **Compound No** | **IC**₅₀ **µ**M | **pIC50** |
|---|---|---|---|---|
| 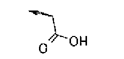 | H | 24 | 1.4 | 5.9 |
| 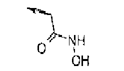 | H | 78 | 3.4 | 5.5 |
| 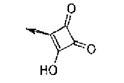 | H | 81 | 12.5 | 4.9 |

It can be seen from Table 1 that the best inhibitor of IDE is the compound in which R₉ is ―COOH, as compared to the compounds in which R₉ is a squaric or a hydroxamic group.

More preferred compounds of formula I-1 are those in which R₉ is COOH or COOMe.

Among the compounds of formula I in which R₉ is ―COR', preferred compounds are those in which, furthermore, R₈ is a (linear C₁-C₆ alkyl)- Ar group with Ar chosen in the group consisting of a phenyl, naphtyl, quinolein, indole, benzimidazole, benzothiazole, pyridine, pyrimidine, or pyrazine group, these Ar groups being optionally substituted with an halogen atom such as Cl, Br, I, F, or a ―CF₃ group, or a linear or branched C₁-C₆ alkyl group, or a -O-(C₁-C₆ alkyl) group.

These compounds have the following formula I-2: 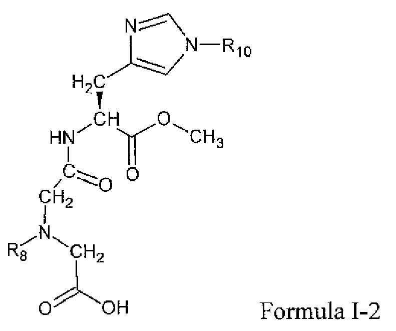 wherein:
- R represents a histidyl derivative of formula II in which R₁ has the following formula: 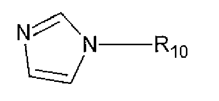 and,
- R₈ and R₁₀ are chosen among the following groups:

| **R**₈ | **R**₁₀ |
|---|---|
| 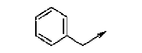 | H |
| 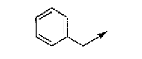 | Trityl |
| H | H |
| CH3 | H |
| CH3 | Trityl |
| 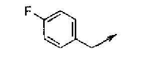 | H |
| 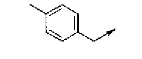 | H |
| 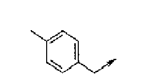 | Trityl |
| 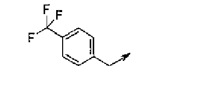 | Trityl |
| 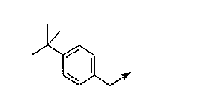 | H |
| 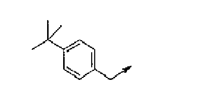 | Trityl |
| 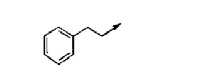 | H |
| 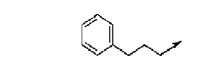 | H |
| 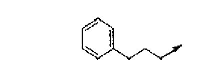 | Trityl |
| 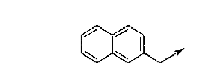 | H |
| 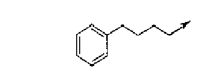 | H |
| 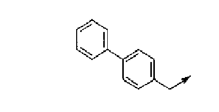 | H |
| 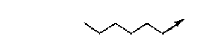 | H |
| 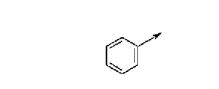 | H |
| 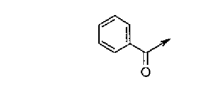 | H |
| 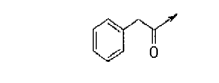 | H |
| 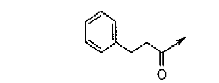 | H |
| 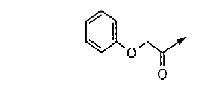 | H |
| 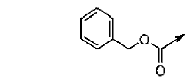 | H |
| 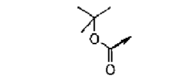 | H |
| 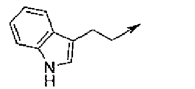 | H |

The *in vitro* inhibition activities of these compounds have been measured by the same method as used for the compounds of formula I-1.

They are reported in the following Table 2.

**Table 2**

| **R**₈ | **R₁₀** | **Compound No** | **IC**₅₀ **µM** | **pIC50** |
|---|---|---|---|---|
| 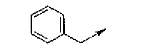 | H | 24 | **1.4** | **5.9** |
| 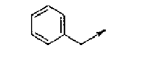 | Trityl | 38 | **0.5** | **6.3** |
| CH3 | Trityl | 31 | **1.7** | **5.8** |
| 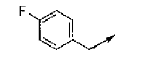 | H | 32 | **2.5** | **5.6** |
| 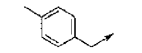 | H | 70 | **1.7** | **5.8** |
| 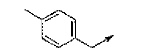 | Trityl | 41 | **0.9** | **6.0** |
| 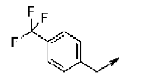 | Trityl | 42 | **39** | **4.4** |
| 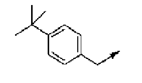 | H | 71 | **2.9** | **5.5** |
| 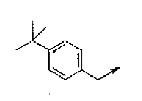 | Trityl | 43 | **22** | **4.6** |
| 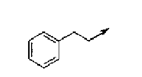 | H | 29 | **0.6** | **6.2** |
| 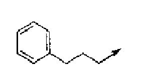 | H | 69 | **0.6** | **6.2** |
| 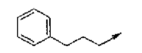 | Trityl | 40 | **1.3** | **5.9** |
| 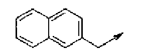 | H | 33 | **1.2** | **5.9** |
| 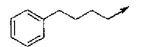 | H | 50 | **0.3** | **6.5** |
| 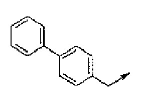 | H | 51 | **4.4** | **5.4** |
| 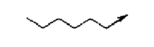 | H | 73 | **0.4** | **6.4** |
| 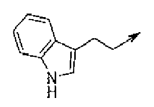 | H | 76 | **1.19** | **5.9** |

The influence of the stereochemistry of the chiral carbon of the compounds of formula I has been studied.

Accordingly, other preferred compounds of the invention have the formula I in which R₉ is a -COOH group, R₂ is H, R₃ is -(C=O)OCH₃, R represents a histidyl derivative of formula II in which R₁ is an optionally substituted imidazole group of the following Formula B:

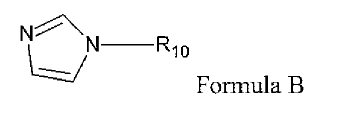

They have the following formula I-3: 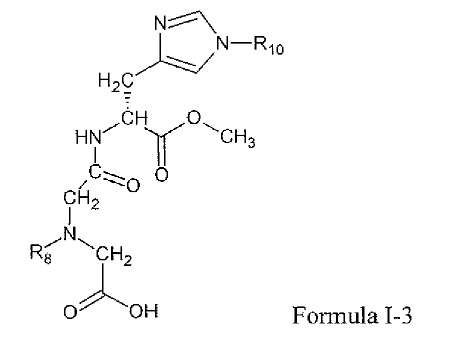 in which R₈ and R₁₀ are chosen among the following groups:

| **series** | **R**₈ | **R**₁₀ |
|---|---|---|
| **L** | 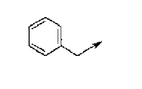 | H |
| **D** | | |
| **L** | 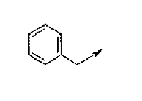 | Trityl |
| **D** | | |
| **L** | 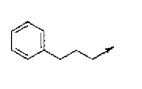 | H |
| **D** | | |
| **L** | 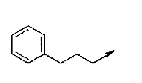 | Trityl |
| **D** | | |
| **L** | 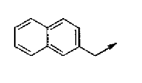 | **H** |
| **D** | | |
| **L** | 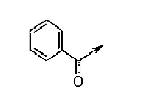 | **H** |
| **D** | | |
| **L** | 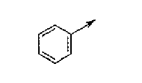 | **H** |
| **D** | | |

The *in vitro* inhibition activities on IDE of these compounds have been measured with the same protocol as used for compounds of formula I-1 and formula I-2 and are reported in the following Table 3.

**Table 3**

| **series** | **R**₈ | **R**₁₀ | **Compound No** | **IC₅₀ µM** | **pIC50** |
|---|---|---|---|---|---|
| **L** | 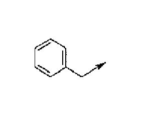 | H | 24 | **1.4** | **5.9** |
| **D** | | | 68 | **1.4** | **5.9** |
| **L** | 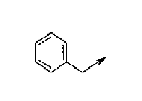 | Trityl | 38 | **0.5** | **6.3** |
| **D** | | | 39 | **0.8** | **6.1** |
| **L** | 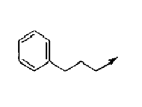 | H | 69 | **0.6** | **6.2** |
| **D** | | | 72 | **0.4** | **6.4** |
| **L** | 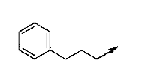 | Trityl | 40 | **1.3** | **5.9** |
| **D** | | | 44 | **8** | **5.1** |
| **L** | 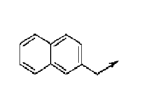 | H | 33 | **0.3** | **6.5** |
| **D** | | | 34 | **0.2** | **6.7** |

Still among the compounds of formula I in which R₉ is COR', preferred compounds are compounds of the following formula I-4: 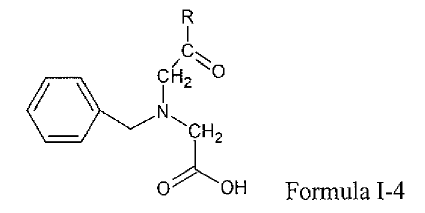 in which R is chosen among the following groups and in which the arrow designates the point of bonding of R to the corresponding carbon atom of the compound of Formula I-1:

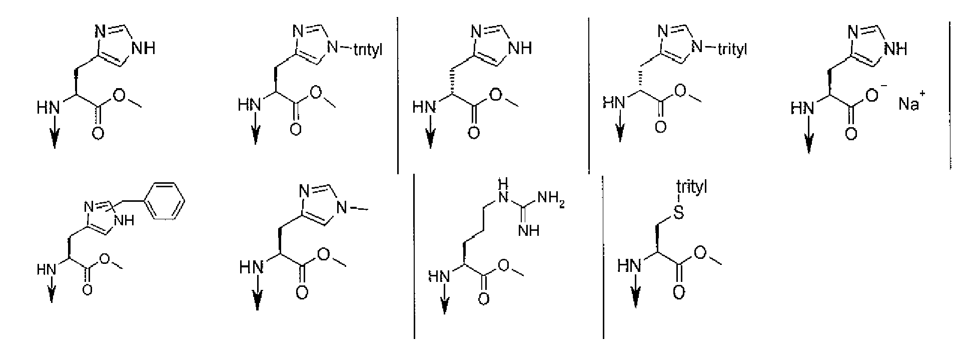

The inhibition activity of these compounds of formula I-4 have been measured.

Following Table 4 indicates the *in vitro* mean inhibition concentration (IC₅₀) in µM measured according to the same protocol as compounds of formulae I-1 to I-3.

**Table 4**

| **R** | **Compound No** | **IC₅₀ᵢₙ µM** | **pIC50** |
|---|---|---|---|
| 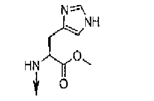 | 24 | **1.4** | **5.9** |
| 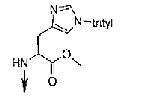 | 68 | **0.5** | **6.3** |
| 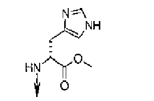 | 39 | **1.4** | **5.9** |
| 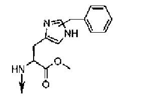 | 30 | **6.3** | **5.2** |
| 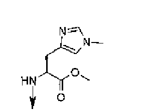 | 32 | **3.7** | **4.4** |
| 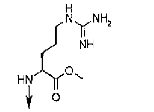 | 37 | **36.3** | **4.4** |
| 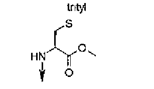 | 35 | **36.5** | **4.4** |

As it can be seen from Table 4, the best inhibitors are those in which R₁ is 4-imidazole group which is not substituted or N- substituted, preferably in tau position, by a methyl, benzyl or trityl group, and those in which R₃ is ―CONR₅R₆ group in which R₅ and R₆ are identical or different and are independently chosen in the group consisting of H, a C₁-C₇ alkyl group, a C₁-C₇ cycloalkyl group, and a benzyl group.

Furthermore, as it can be seen from Table 2, the best inhibitors are those in which R₈ is a (linear C₃ or C₄ alkyl)-phenyl group.

These compounds have the following formula I-5: 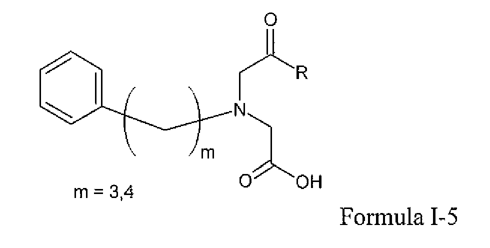 in which m = 3 or 4 and R is defined in following Table 5 and their *in vitro* mean inhibitory concentrations measured according to the same protocol as for compounds of formulae I-1 and 1-4 are reported in following table 5:

**Table 5**

| -R | m | Compounds No | IC₅0 (µM) | pIC₅₀ |
|---|---|---|---|---|
| 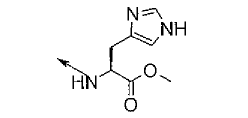 | 3 | 69 | 0.6 | 6.2 |
| 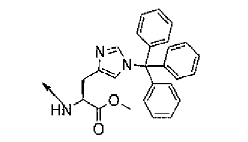 | 3 | 40 | 1.3 | 5.9 |
| 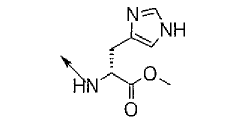 | 3 | 72 | 0.4 | 6.4 |
| 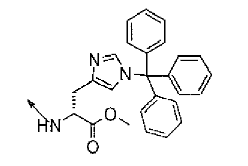 | 3 | 44 | 8.0 | 5.1 |
| 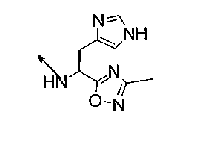 | 3 | 57 | 0.468 | 6.3 |
| 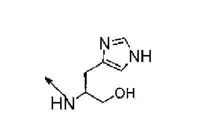 | 3 | 53 | 1.6 | 5.8 |
| 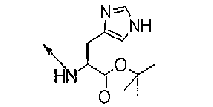 | 3 | 75 | 0.4 | 6.4 |
| 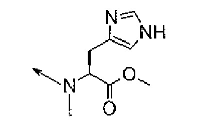 | 3 | 56 | 7.98 | 5.1 |
| 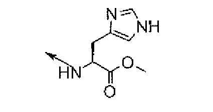 | 4 | 50 | 0.3 | 6.5 |

Also, as it can be seen from Tables 1 to 5, preferred compounds of formula I are those in which R₂ is H.

As shown in Tables 1 to 5, more preferred compounds of the invention are those indicated in Table 5 plus the compound 80.

The most preferred compound is compound 80 having the following formula I-6:

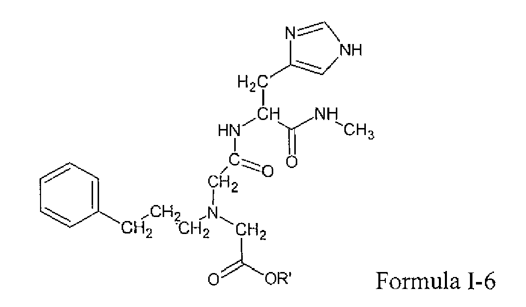

The compounds of formula I of the invention may be as well inhibitors as activators of IDE according to the substrate.

For example, the compounds **80** and **24** are inhibitors of IDE on a substrate ATTO 655-Cys-Lys-Leu-Val-Phe-Phe-Ala-Glu-Asp-Trp whereas they are activators of IDE when the substrate is insulin.

Because the compounds of formula I are modulators of IDE activity (i.e inhibitors or activators of IDE according to the substrate of IDE), they can be used as medicaments for the treatment of diabetes, Alzheimer's disease, cancers, acromegaly, growth hormone deficit, obesity, hypertension, acute heart failure, cardiac hypertrophy, inflammation, infectious diseases and pain, according to the substrate of IDE alone or in association with other active compounds.

The following Table 6 summarizes the diseases or disorders which can be treated with the compounds of formula I, according to the substrate of IDE. In all cases, according to the stage of the disease, activator or inhibitor may be useful as hormone saving or secreting organ saving drug (31).

**Table 6**

| **Substrate of IDE** | **Therapeutic application** |
|---|---|
| insulin | Diabetes |
| Beta-amyloid | Alzheimer's disease |
| Glucagon | Diabetes |
| IGF-2 et IGF-1 | Cancer, Diabetes |
| Somatostatin | Acromegaly, growth hormone deficit, Alzheimer's disease, diabetes, cancer |
| Amylin | Obesity, Diabetes |
| ANP, BNP | Hypertension, acute heart failure, cardiac hypertrophy |
| TGF alpha | Inflammation, Infectious diseases |
| Beta endorphin | pain |

Therefore, the instant invention also relates to pharmaceutical compositions, characterized in that they comprise a compound of formula I, as defined hereabove and at least one pharmaceutically acceptable vehicle.

The pharmaceutical acceptable vehicle may be any vehicle convenient for enteral or parenteral administration.

For instance, said vehicle may be: water, gelatine, gum Arabic, lactose, starch, magnesium stearate, talc, vegetable oils, and the like. Additional additives may also be incorporated in the pharmaceutical composition such as flavouring agents, preservatives, stabilizers, emulsifying agents, buffers and the like.

The pharmaceutical composition may be in a solid form for oral administration, such as tablets, capsules, pills, powders, granules and the like or in a liquid form for oral administration such as solutions, syrups, suspensions, and the like; they may also be adapted for a parenteral administration such as sterile solutions, suspensions or emulsions.

The compounds of the invention, due to their binding to the exosite of IDE, are appropriate for screening compounds which also bind to the exosite of IDE which will then be evaluated as to their ability to modulate (to inhibit or to activate) IDE.

Therefore, the instant invention also relates to a method of identifying or screening a compound able to modulate IDE activity, said method comprising:
(i) contacting IDE with a labelled reference compound of Formula I, having a pIC₅₀ >6 and preferably >7 and a compound to be tested and
(ii) measuring the change in strength of the signal emitted by the label, preferably a fluorescence signal (fluorescence polarisation, fluorescence intensity or fluorescence correlation spectroscopy), a decrease in the strength of said signal being correlated to the binding of said compound to be tested to the exosite of IDE more potently than the reference compound.

The reference compound is preferably compound 80; the reference compound may be labelled with a radioactive molecule (tritium, C¹⁴ or Iodine 131 or 125) or a fluorophore (with λexc/em > 380nm like for example bodipy probes from Invitrogen.Inc).

In order that the invention be better understood, examples of production methods are given hereafter for none limitative and illustrative purposes, only.

However the production method of the compounds of the present invention is not limited to these methods. Also, it is possible to previously protect, as necessary, the functional groups other than those involved in the reaction mentioned, and to deprotect them at a later stage. The production of the exemplified compounds described proceeds mainly from pathways described in following (Scheme). For example, compounds can be obtained either by nucleophic opening of anhydrides with amines when R₉ is a carboxylic acid, or by activation of the adequate carboxylic acid and subsequent reaction with an amine to form the amide bond. Other pathways can also be used as described on the right part of scheme 1. The obtained product can be further derivatized or deprotected when needed.

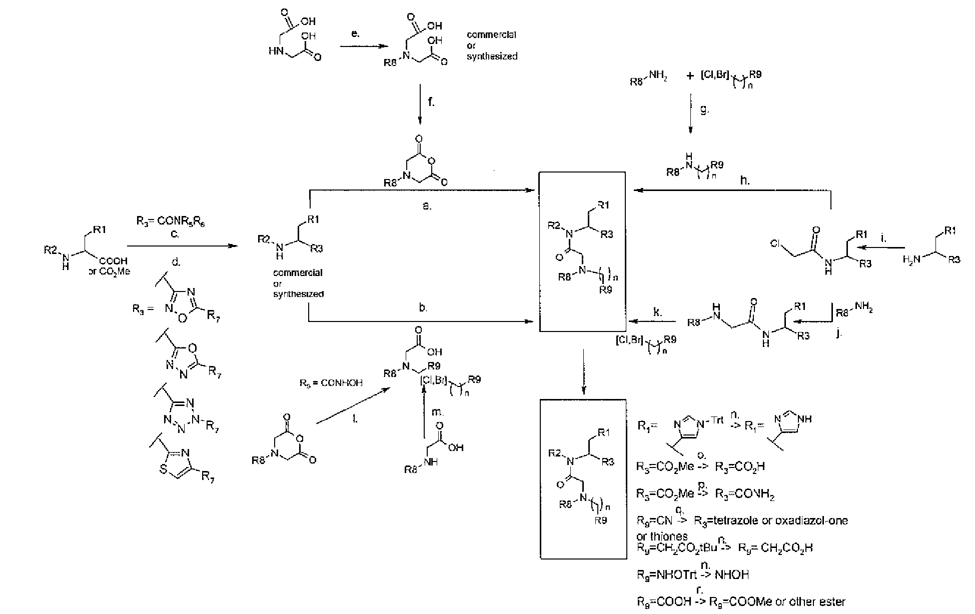

**Scheme 1:** Example of production method of compounds of the invention with R₁ to R₉ as defined above or previously protected as necessary.

### Examples of reactions and reaction conditions:

***a.*** *nucleophilic opening of an anhydride by amines to give amides: amine, anhydride, diisopropylamine in DCM, room temp.;*
***b.*** *nucleophilic substitution of an activated carboxylic acid by amines to give amides:carboxylic acid, HOBt, EDCI in DMF, amine;*
***c.*** *nucleophilic substitution of an activated carboxylic acid by amines to give amides: carboxylic acid, HOBt, EDCI, HNR₅R₆ in DMF or aminolysis of an ester to give amides: carboxylic ester, HNR₅R₆;*
***d.*** *synthesis of 1,2,4-oxadiazoles : nucleophilic substitution of an activated carboxylic acid by amidoxime then dehydration: carboxylic acid, HOBt, TBTU in DMF HO-N=CR₇-NH₂ (27); synthesis of tetrazoles: i. carboxylic acid, N,N'carboxydiimidazole, DMF, then ii. NH₃ to give amide, then iii. dehydration into nitrile then formation of the tetrazole ring iv. ZnBr₂, N₃-R₇ (28); synthesis of thiazoles: i. activation of carboxylic acid : carboxylic acid, N,N'carboxydiimidazole, DMF, then ii. Amide formation: NH3, iii. Thioamide formation: Laweson's reagent then iv. Alkylation and cyclisation into thiazoles: Cl-CH₂*-*CO-R₇ (29); synthesis of 1,3,4-oxadiazoles by activation of carboxylic acid and further nucleophilic substitution by hydrazide and cyclocondensation: carboxylic acid, phosphorous oxychloride, H₂N-NH-CO-R₇ (30).*
***e.*** *Synthesis of N-substituted iminodiacetic acid : alkylation of iminodiacetic acid by halide, diisopropylamine in MeOH or acylation of iminodiacetic acid by chloroformiate or acylchloride in DMF.*
***f.*** ***Cyclodehydration of N-substituted iminodiacetic acid into corresponding anhydride:** TFAA or DCC;*
***g**. Alkylation of amine by halide, Na₂CO₃, KI, DMF;*
***h.*** *Alkylation of amine by halide Na₂CO₃, KI, DMF;*
***i**. acylation of amine with acylchloride CICOCH₂Cl or equivalent, NaHCO₃, DCM to give corresponding amide.*
***j.*** *Alkylation of amine by halide Na₂CO₃, KI, DMF;*
***k.*** *Alkylation of amine by halide Na₂CO3, KI, DMF;*
***l**. Nucleophilic opening of anhydride : diisopropylamine, H₂N-O-Trt to give hydroxamate, , or alcohol R-OH to give ester;*
***m.*** *Alkylation of amine by halide, NaHCO₃, DCM;*
***n.*** *Deprotection of trityle groups or tert-butyl groups : triisopropylsilane, TFA , TIS,DCM;*
***o.*** *Saponification of methyl ester into the corresponding carboxylic acid: NaOH H₂O, MeOH*
***p.*** *Aminolysis of methyl esters into amide: methyl ester, ammonia in dioxane ;*
***q.*** *Nucleophilic substitution of nitrile to give tetrazole : sodium azide, ammonium chloride, DMF, 90°C or 1,2,4-oxadiazol-5-one or 1,2,4-oxadiazol-5-thione i.hydroxylamine, diisopropylamine, ii N,N'-carbonyldiimidazole or N,N'-thiocarbonyldiimidazole.;*
***r.*** *esterification of carboxylic acid: carboxylic acid,SOCl₂, corresponding alcohol, heat.*

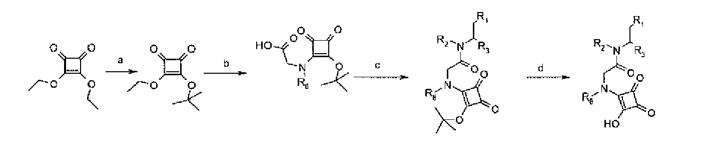

**Scheme 2.** *Reactants and conditions :* a) Transesterification of diethyl squarate to obain the corresponding tert-butyl ether: potassium *tert*-butylate 1M in tetrahydrofuran, diethyl squarate, THF, 4°C, 15 min b) nucleophilic substitution of ethyl ether by an amino-acid to give the enamine : R₈-N-CH₂COOH, NEt₃, MeOH, overnight, room temp. c) amide formation from activated carboxylic acid and amine :EDCI, HOBt, DIEA, R₂NH-CH(R₃)-CH₂-R₁, CH₂Cl₂, room temp., overnight d) Deprotection of tert-butyl group to give the corresponding squaric acid derivative : TFA/ DCM 50/50, 4°C, 30 min.

In scheme 1 and 2 above, R₁, R₂, R₃, R₇, R₈ and R₉ have the same meanings as those defined for formulae I and II.

### Example 1: synthesis of iminodiacetic acid derivatives (1-12)

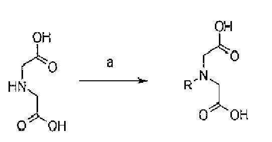

**Scheme 3:** *Reactants and conditions: a)* R-Br, MeOH, DIEA or TEA, room temp., 2-12 h To a solution of iminodiacetic acid hydrochloride (2 mmol) in MeOH (10 mL) were added NEt₃ (6 mmol) and the bromide derivative (2 mmol). The mixture was stirred overnight at room temperature or refluxed. The crude product was purified by preparative HPLC.

**N-(4-fluorobenzyl)iminodiacetic acid acid (1)** was obtained as a white powder (55 %). Purity 100%; ¹H NMR (DMSO-*d6*) δ ppm: 7.30 (m, 2H), 7.12 (m, 2H), 3.77 (s, 2H), 3.31 (s, 4H). LC t_{R}= 1.93 min, MS (ESI+): m/z= 242(M+H)⁺.

**N-(phenethyl)iminodiacetic acid (2)** was obtained as a white powder (36 %). Purity 100%; ¹H NMR (DMSO-*d6*) δ ppm: 7.22 (m, 5H), 3.46 (s, 4H), 2.86 (m, 2H), 2.70 (m, 2H). LC t_{R}= 1.12 min, MS (ESI+): m/z= 238(M+H)⁺.

**N-(4-methylbenzyl)iminodiacetic acid (3)** was obtained as a white powder (31 %). Purity 90%; ¹H NMR (DMSO-*d6*) δ ppm: 12.27 (s, 3H), 3.34 (s, 2H), 7.10 (d, *J*= 7.8 Hz, 2H), 7.20 (d, *J=* 7.8 Hz, 2H) LC tᵣ = 2.38 min; MS (ESI+) : m/z = 238 (M+H)⁺. **N-(3-phenylpropyl)iminodiacetic acid (4)** was obtained as a white powder (27 %). Purity 99%; ¹H NMR (DMSO-*d6*) δ ppm: 1.67 (dt, *J* = 7.5Hz, 2H), 2.56 (t, *J*= 7.5Hz, 2H), 2.65 (t, J = 7.5Hz, 2H), 3.41 (s, 4H), 7.13-7.26 (m, 5H). LC tᵣ = 2.76 min; MS (ESI+): m/z = 252 (M+H)⁺.

**N-(naphthalen-2-ylmethyl)iminodiacetic acid (5)** was obtained as a white powder (57 %). Purity100%; ¹H NMR (DMSO-*d6*) δ ppm: 7.89-7.85 (m, 3 H), 7.77 (s, 1H), 7.57 (dd, *J* = 1.5 and 8.4 Hz, 1H), 7.51-7.45 (m, 2H). LC tᵣ = 3.13 min; MS (ESI+): m/z = 274 (M+H)⁺.

**N-(4-trifluoromethyl-benzyl)iminodiacetic acid (6)** was obtained as a white powder (43 %). Purity 99%;¹H NMR (DMSO-*d6*) δ ppm: 3.42 (s, 4H), 3.91 (s, 2H), 7.58 (d, *J=* 7.8Hz, 2H), 7.68 (d, J= 7.8Hz, 2H). LC tᵣ = 3.17 min; MS (ESI+): m/z = 292 (M+H)⁺. **N-(4-tert-Butylbenzyl)iminodiacetic acid (7)** was obtained as a white powder (43 %). Purity 85%; ¹H NMR (DMSO-*d6*) δ ppm: 1.28 (s, 9H), 3.37 (s, 4H), 3.76 (s, 2H), 7.23 (d, J= 8.4Hz, 2H), 7.34 (d, J= 8.4Hz, 2H) LC tᵣ = 3.88 min; MS (ESI+) : m/z = 280 (M+H)⁺. **N-(4-phenylbutyl)iminodiacetic acid (8)** was obtained as a white powder (76 %). Purity : 99 %; ¹H NMR (DMSO-d6) δ ppm : 7.26-7.12 (m, 5H), 3.39 (s, 4H), 2.65 (t, J = 7.1Hz, 2H), 2.55 (t, J = 7.1Hz, 2H),1.54 (m, 2H), 1.39 (m, 2H); LC : t_{R}= 3.59 min; MS (ESI+): m/z=266 (M+H)⁺.

**N-(n-hexyl)iminodiacetic acid (9)** Purity 100%; LC : tᵣ = 2.9 min ; MS (ESI+) : m/z = 218 (M+H)⁺.

***N-*(4-phenyl-benzyl)iminodiacetic acid (10)** was obtained from the commercial 4-phenyl benzyl chloride. Purity : 59% ; LC : tᵣ = 3.73 min; MS (ESI+) : m/z = 300 (M+H)⁺.

***N-*(2-(3-indolyl)ethyl)iminodiacetic acid (11)** was obtained as an oil (58 %). Purity: 98% ;¹H NMR (DMSO-*d6*) δ ppm : 10.77 (sl, NH), 7.49 (d, J= 7.7Hz, 1H), 7.31 (d, J= 8.04Hz, 1H), 7.16 (d, J = 2.16Hz, 1H), 7.04 (td, *J =* 1.1 Hz and J = 7.1Hz and *J* = 8.04Hz, 1H), 6.94 (td, J = 1.1 Hz and *J =* 7.1Hz and *J* = 7.7Hz, 1H), 3.50 (s, 4H), 2.88 (m, 4H); LC : tᵣ = 3.28min; MS (ESI+) : m/z = 277 (M+H)⁺.

***N*-2-(4-pyridinyl)iminodiacetic acid (12)** Purity : 98% ; ¹H NMR (DMSO-*d6*) δ ppm : 8.48 (d, J = 5.7 Hz, 2H), 7.29 (d, J = 5.7 Hz, 2H), 3.83 (s, 2H), 3.35 (s, 4H); LC : tᵣ = 0.87min; MS : (ESI-) : m/z = 223 (M-H)⁺.

### Example 2: synthesis of other carboxylic acids (13-16)

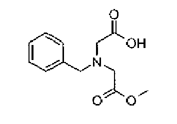

**(Benzyl-methoxycarbonylmethyl-amino)-acetic acid (13)** *N-*(benzyl)iminodiacetic acid (2 mmol) was dissolved in trifluoracetic anhydride 2% in acetic anhydride (5 mL). The reaction mixture was stirred for 4 hours at room temperature or 70°C if product was not soluble at room temperature and then evaporated. The residue was dissolved in DMF (2mL). Methanol (2.0 mmol) and DIEA (660µL) were added. The mixture was stirred overnight at room temperature under argon. The solvent was evaporated under reduced pressure. The crude product was dissolved in DCM and washed with water. Purity : 97% ; MS : (ESI+): m/z = 238 (M+H)⁺.

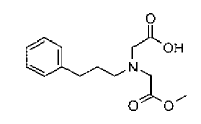

**[Methoxycarbonylmethyl-(3-phenyl-propyl)-amino]-acetic acid (14)** (2 mmol) was dissolved in trifluoracetic anhydride 2% in acetic anhydride (5 mL). The reaction mixture was stirred for 4 hours at room temperature or 70°C if product was not soluble at room temperature and then evaporated. The residue was dissolved in DMF (2mL). Methanol (2.0 mmol) and DIEA (660µL) were added. The mixture was stirred overnight at room temperature under argon. The solvent was evaporated under reduced pressure. The crude product was dissolved in DCM and washed with water. Purity : 97% ; MS : (ESI+) : m/z = 2.66 (M+H)⁺. 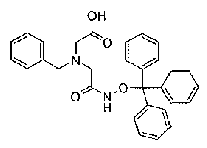 **[Benzyl-(trityloxycarbamoyl-methyl)-amino]-acetic acid (15)** : *N-*(benzyl)iminodiacetic acid (2 mmol) was dissolved in trifluoracetic anhydride 2% in acetic anhydride (5 mL). The reaction mixture was stirred for 4 hours at room temperature or 70°C if product was not soluble at room temperature and then evaporated. The residue was dissolved in DMF (2mL). O-trityl hydroxylamine (550mg, 2.0 mmol) and DIEA (660µL) were added. The mixture was stirred overnight at room temperature under argon. The solvent was evaporated under reduced pressure. The crude product was dissolved in DCM and washed with water. **(15)** was obtained as an oil (900mg, 93%) and was used without further purification.

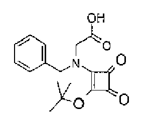

**[Benzyl-(2-*te*r*t*-butoxy-3,4-dioxo-cyclobut-1-enyl)-amino]-acetic acid (16)** :A solution of potassium *tert*-butylate (17.6 mL, 17.6 mmol, 1 eq.), 1M in tetrahydrofuran was added at once to a solution of diethyl squarate (3 g, 17.6 mmol, 1 eq.) in 50 mL of tetrahydrofuran at 4°C. After 15 minutes, the reaction was acidified with 1N HCl (1 eq.) to pH 2-3. The mixture was extracted three times with ether. The combined organic layers were washed with sat. aq. NaCl, dried over MgSO₄ and solvent was evaporated under reduced pressure. The crude product was purified by silica gel chromatography (eluent: cyclohexane/AcOEt (97/3)) to give the desired product (1.95g, 55%). ¹HNMR (300 MHz, CDCl₃, δ ppm: 4.762 (q, *J*=7.2Hz, 2H), 1.601 (s, 9H), 1.480 (t, *J*= 7.2 Hz, 3H). LC t_{R}=5.061 min. *m*/*z*: [M-²⁴*t*Bu+2H]⁺=143. [M-tBu-Et+2H]⁺=115 (Pirrung et al. J Org. Chem. 1996, 61, 4527-4531). To a solution of N-benzylglycine hydrochloride (305 mg, 1.51 mmol) and NEt₃ (424 µL, 302 mmol) in MeOH (10 mL), was added ethyl tert-butyl squarate (300 mg, 1.51 mmol). The mixture was stirred at room temperature overnight and MeOH was evaporated. The crude product was purified by preparative HPLC to give **(16)** as a colorless oil (330 mg, 70%). Purity 100%; LC t_{R}=5.28 min, MS (ESI+): m/z=316 (M-H)⁻.

### Example 3: synthesis of amines 17-23

**H-L-Cys(Trt)-OMe (17) as the formiate salt** Fmoc-Cys(Trt)-OH (1g, 1.7 mmol) was activated with oxalyl chloride (175 µL, 2.04 mmol) in dichloromethane (with catalytic DMF) at 0°C. The mixture was stirred 50 minutes, then solvent was evaporated. The residue was dissolved and stirred in methanol during 4 hours. The solvent was evaporated to give Fmoc-Cys(Trt)-OMe as a yellow oil (1.34 g, 100%). Purity 89%;¹H NMR (DMSO-*d6*) δ ppm : 2.48 (dd, *J* = 1.8Hz, 1H), 2.50 (dd, *J*= 1.8Hz, 1H), 3.52 (s, 3H), 3.83 (ddd, *J* = 5Hz, 4Hz, 4.8Hz, 1H), 4.2 (m, 2H+1H), 7.30-7.33 (m, 15H+4H), 7.70 (d, J = 7.5Hz, 2H), 7.88 (m, 2H); LC tᵣ = 9.47 min; MS (ESI+) : m/z = 622 (M+Na)⁺. To a stirred solution of piperidine/DMF (20%) was added to Fmoc-Cys(Trt)-OMe (1 g, 1.7 mmol). The mixture was stirred for 1.5 hours at room temperature. The solvent was evaporated to give **(14)**. Purity 6 1 %; ¹HNMR (DMSO-*d6*) δ ppm: 2.26-2.32 (dd, *J*= 11.4Hz, J = 6.6Hz, 1H), 2.37-2.43 (dd, *J* = 12Hz, 6.3Hz and 6.6Hz, 1H), 3.21 (dd, J = 6.3Hz and 6.6Hz, 1H), 3.57 (s, 3H), 7.29-7.36 (m, 15H). LC tᵣ = 5.16 min; MS (ESI+): m/z = 400 (M+Na)⁺ **H-L-His-*Oi*Bu (18) dihydrochloride**. To a stirred solution of H-L-His-OH (463mg, 2.98mmol) in iBuOH (5mL) was added at 0°C thionyl chloride (2mL). The solution was heated at 60°C for 2 days and the solvent was evaporated. The crude product was used directly in next reaction.

**H-L-His-OiPr (19) dihydrochloride** To a stirred solution of Boc-L-His-OH (1.27 g, 5 mmol) in DCM (30mL) were added iPrOH (5 mL, 65 mmol), DMAP (671mg, 5.5mmol), EDCI (1.05 g, 5.5 mmol) and DIEA (956 µL, 5.5 mmol). The mixture was heated at 50°C 28 hours and washed with a saturated solution of NaHCO₃. The organic layers were dried with MgSO₄ and evaporated. The crude product was purified by preparative HPLC as a colorless oil (832mg, 56%). Purity : 98% ; ¹H NMR (DMSO-*d6*) δ ppm : 7.58 (s, 1H), 7.14 (d, J = 7.5 Hz, 1H), 6.80 (s, I H), 4.83 (sept, *J* = 6.2 Hz, 1H), 4.13 (m, 1H), 2.83 (m, 2H), 1.35 (s, 9H), 1.13 (d, J= 6.3 Hz, 3H), 1.09 (d,J= 6 Hz, 3H); LC : tᵣ = 3.6min; MS (ESI+): m/z = 298 (M+H)⁺. Deprotection was preformed with HClg in dichloromethane during 30 min at room temperature. Solvent was evaporated to give **(16)** (586 mg, 77%). Purity : 98%; ¹H NMR (DMSO-*d6* δ ppm : 9.08 (d, J = 1.2 Hz, 1H), 7.53 (d, J = 1.2 Hz, 1H), 4.93 (sept, *J* = 6.2 Hz, 1H), 4.13 {m, 1H), 2.83 (m, 2H), 1.35 (s, 9H), 1.13 (d, *J* = 6.3 Hz, 3H), 1.09 (d, *J* = 6 Hz, 3H); LC : tᵣ = 0.65 min.

**H-L-His-NMe₂ dihydrochloride (20)** To a stirred solution of Boc-L-His-OH (765mg, 3 mmol) in DMF (20mL) were added dimethylamine.HCl (293mg, 3.6mmol), HOBt (551mg, 3.6mmol, EDCI (690 mg, 3.6mmol)and NEt₃ (1mL, 6.8mmol). The mixture was stirred overnight. The solvent was evaporated. The crude product was solubilised in DCM and washed with a sat.NaHCO₃ The organic layers were dried with MgSO₄ and evaporated. The crude product (198 mg, 0.7mmol) was deprotected with HClg in dichloromethane during 30 min at room temperature. The solvent was evaporated to give (17) as a dihydrochloride salt used directly in next reaction.

**H-L-histidine benzylamide dihydrochloride (21)** To a stirred solution of Boc-L-His-OH (500mg, 1.95 mmol) in DMF (10mL) were added benzylamine (213µL, 1.95mmol), HOBt (264mg, 1.95mmol), EDCI (455mg, 2.38mmol) and DIEA (2mL, 11.7mmol). The mixture was stirred overnight. The solvent was evaporated. The residue was solubilised in DCM and washed with a saturated solution of NaHCO₃ then with brine. The organic layers were dried with MgSO₄ and evaporated. The crude product was purified by preparative HPLC to give the expected compound (428 mg, 64%). Purity : 98% ; ¹H NMR (DMSO-*d6*) δ ppm : 8.28 (t, J = 6Hz, 1H), 7.57 (s, 1H), 7.29-7.11 (m, 5H), 6.98 (d, J = 8.1Hz, 1H), 6.79 (s, 1H), 4.25 (d, J = 6Hz, 2H), 4.18 (m, 1H), 2.89-2.75 (m, 2H), 1.36 (s, 9H); LC : tᵣ = 3.87min; MS (ESI+) : m/z = 345 (M+H)⁺. Intermediate (428 mg, 1.2 mmol) was deprotected in presence of HClg in dichloromethane during 30 min at room temperature. Solvent was evaporated to give **(18)** used directly in next reaction Purity : 98% ; LC : tᵣ = 4.32min; MS : (ESI+) : m/z = 245 (M+H)⁺.

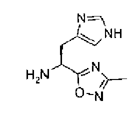

**(S)-2-(1*H*-Imidazol-4-yl)-1-(3-methyl-[1,2,4]oxadiazol-5-yl)-ethylamine (22)** To a stirred solution of Boc-L-His-OH (510mg, 2mmol) in DMF (10mL) were added TBTU (702mg, 2.2mmol) and DIEA (1mL, 6mmol). The resulting mixture was stirred for 5minutes. Acetamidoxime (150mg, 2mmol) was added and the mixture was stirred for 4.5 h. The solution was refluxed 1.5 h. The solvent was evaporated and the residue was dissolved in AcOEt, washed with a sat. aq. NaHCO₃. The organic layers were dried over MgSO₄ and evaporated. The crude product was purified by preparative HPLC to give an oil (426mg, 73%). Purity : 98% ; ¹H NMR (DMSO-*d6*) δ ppm : 8.19 (s, 1H), 7.67 (d, *J* = 7.8Hz, 1H), 7.54 (s, 1H), 6.76 (s, 1H), 5.03 (m, 1H), 3.02 (m, 2H), 2.29 (s, 3H), 1.35 (s, 9H); LC : tᵣ = 3.56 min; MS (ESI+) : m/z = 294 (M+H)⁺. The intermediate (416 mg, 1.42 mmol) was deprotected in presence of HCl*g* in dichloromethane during 30 min at room temperature. Solvent was evaporated to give the expected product as a dihydrochloride salt used directly in next reaction. Purity: 98% ; LC : tᵣ = 0.66min; MS (ESI+ : m/z = **194** (M+H)⁺.

**H-L-His(1-Trt)-NHMe (23) as the formiate salt** To a stirred solution of H-L-His(Trt)-OMe (2.5 g, 5.5mmol) in methanol (10 mL) were added 4mL of a solution of methylamine in ethanol (33%)The mixture was refluxed overnight. The solvent was evaporated and the crude product was precipitated in water and filtrated. The crude product was purified by preparative HPLC to give the compound as a white powder (1.43g, 63%). Purity : 74% ; ¹H NMR (DMSO-*d6*) δ ppm : 8.30 (s, 1H), 7.95 (q, J = 4.8Hz, 1H), 7.06-7.43 (m, 15H), 6.63 (s, 1H), 3.53 (dd, J = 5.7Hz and J = 7.2Hz, 1H), 2.77 (dd, J = 5.7Hz and *J* = 14.4Hz, 1H), 2.63 (dd, *J* = 5.7Hz and J = 14.4Hz, 1H), 2.54 (d, *J* = 4.8Hz, 3H); LC : tᵣ = 4.79min; MS (ESI+) : m/z = 411 (M+H)⁺

### Example 4: Synthesis of final compounds according to the invention (24-59) Nucleophilic opening of anhydride is exemplified in scheme 3 (24-59)

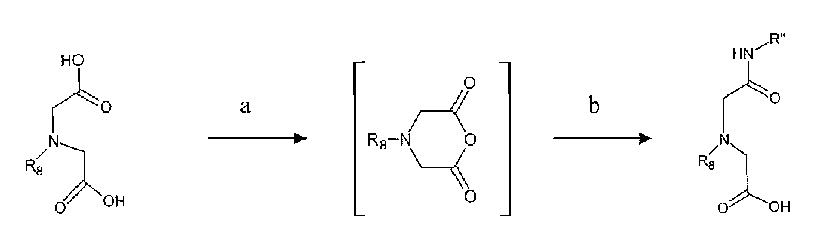

**Scheme 4 :** *Reactants and conditions :* a) trifluoroacetic anhydride 2% in acetic anhydride, 20-70°C, 5h, 100%. b) amine, anh. DIEA, anh.DMF, Argon, room temp., overnight. The diacid (5 mmol) was dissolved in trifluoracetic anhydride 2% in acetic anhydride (5 mL). The reaction mixture was stirred for 4 hours at room temperature or 70°C if product was not soluble at room temperature and then evaporated. To a stirred solution of amine (5 mmol) in DMF (20 mL) were added DIEA (2.6mL) and the anhydride acid (5mmol). The mixture was stirred overnight at room temperature and the solvent was evaporated. The crude product was purified by preparative HPLC to yield compound.

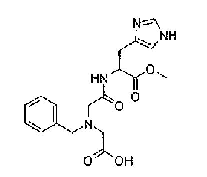

**(S)-2-[2-(Benzyl-carboxymethyl-amino)-acetylamino]-3-(1*H*-imidazol-4-yl)-propionic acid methyl ester (24) as the formiate salt** was obtained as a white powder from N-benzyl-iminodiacetic acid and H-L-His-OMe (17%). Purity 100%; ¹H NMR (DMSO-*d6*) δ ppm: 2.99 (d, *J=* 6.3Hz, 2H), 3.23 (s, 2H), 3.27 (s, 2H) , 3.59 (s, 3H), 3.71 (d, *J*=13.4Hz, 1H), 3.77 (d, *J=*13.4Hz, 1H), 4.65 (m, 1H) , 7.09 (d, *J*=1.1 Hz, 1H) , 7.30 (m, 5H) , 8.26 (d, *J=*1.1 Hz, 1H), 8.34 (d, 1H, NH); LC t_{R}= 2.30 min, MS (ESI+): m/z= 375 (M+H)⁺.

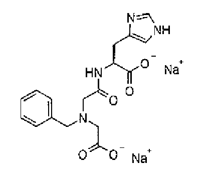

**(S)-2-[2-(Benzyl-carboxymethyl-amino)-acetylamino]-3-(1*H*-imidazol-4-yl)-propionic acid disodium salt (25).** To a stirred solution of **(24)** (125mg, 0.30mmol) in MeOH (3mL) were added NaOH (36mg, 0.90mmol) and 50µL of distilled water. The mixture was stirred overnight at room temperature. The solvent was evaporated. The product is obtained as a white solid (162.4mg, 99%).Purity 89%; ¹H NMR (MeOD) δ ppm: 7.45 (d, *J=* 0.9 Hz, 1H), 7.33-7.21 (m, 5H), 6.81 (s, 1H), 4.52 (dd, *J*= 4.5 and 7.2Hz, 1H), 3.78 (d, *J=* 13.2Hz, 1H), 3.66 (d, *J=* 13.2Hz, 1H). 3.25 (m, 2H), 3.19 (dd, J = 11.1 and 15.6 Hz, 1H), 3.13 (d, *J*=16.5 Hz, 1H), 3.09 (dd, *J=* 7.8 and 15.3 Hz, 1H) 3.05 (d, *J*=16.5 Hz, 1H). LC t_{R}=1.86 min, MS (ESI+): m/z=361 (M+H)⁺.

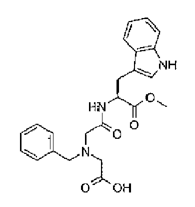

**(S)-2-[2-(Benzyl-carboxymethyl-amino)-acetylamino]-3-(1*H*-indol-3-yl)-propionic acid methyl ester (26)** To a stirred solution of H-L-Trp-OMe dihydrochloride (498mg, 1.97mmol) in DMF (19.7mL) were added DIEA (1.18mL) and N-benzyliminodiacetic anhydride acid (366.9mg, 1.79mmol) in DMF (3.58mL). The mixture was stirred during an hour at room temperature and the solvent was evaporated. The crude product was dissolved in MeOH and HCl (0.1N). The crude product was purified by preparative HPLC to yield compound as a white solid (150.6mg, 18%).Purity 99%; ¹H NMR (DMSO-*d6*) δ ppm: 3.14-3.27 (m, 6H), 3.59 (s, 3H), 3.62 (d, *J*=13.2, 1H), 3.68 (d, *J=13.2,* 1H),4.60 (dd, *J*=7.2 and 15.0 Hz, 1H), 6.98 (td, *J* = 1.2 and 8.1 Hz, 1H), 7.08 (td, *J =* 1.2 and 7.8 Hz, 1H), 7.08-7.10 (m, 2H), 7.16 (d, *J*=2.1Hz, 1H), 7.20-7.22 (m, 3H), 7.36 (d, *J*=8.1 Hz, 1H), 7.48 (d, *J*=7.8Hz, 1H), 8.10 (d, *J*=7.8Hz, 1H), 10.9 (s, 1H), ¹³C NMR (DMSO-*d6*) δ ppm: 172.8, 172.6, 170.7, 138.4, 136.7, 128.8, 128.2, 127.1, 124.2, 121.7, 119.2, 118.2, 111.9, 109.3, 58.0, 57.2, 54.2, 52.4, 52.1, 27.4. LC t_{R}= 4.42 min, MS (ESI +): m/z = 424 (M+H)⁺.

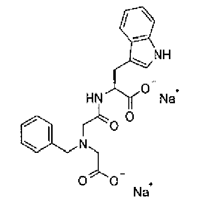

**(S)-2-[2-(Benzyl-carboxymethyl-amino)-acetylamino]-3-(1*H*-indol-3-yl)-propionic acid (27).** To a stirred solution of (S)-2-[2-(Benzyl-carboxymethyl-amino)-acetylamino]-3-(1*H*-indol-3-yl)-propionic acid methyl ester (70mg, 0.15mmol) in MeOH (3mL) were added NaOH (18mg, 0.45mmol) and some drops of distilled water. The mixture was stirred during one night at room temperature. Then the solvent was evaporated. The crude product is a white solid (78.6mg, quantitative). Purity 100%; ¹H NMR (MeOD) δ ppm: 2.93 (d, *J=*16.5Hz, 1H), 2.98 (d, *J=*16.5Hz, 1H), 3.15 (d, *J*=16.5Hz, 1H), 3.24 (d, *J*=16.5Hz, 1H), 3.25 (d, *J*=7.2Hz, 1H), 3.43 (m, 1H), 3.46 (d, *J=*12.9Hz, 1H), 3.59 (d, *J=* 12.9Hz, 1H), 4.60 (dd, *J*=4.8 and 7.2 Hz), 6.91-7.02 (m, 1H + 1H), 7.05-7.16 (m, 5H +1H), 7.22 (m, 1H), 7.62 (m, 1H). LC t_{R}=3.85 min, MS (ESI+): m/z=410 (M+H)⁺.

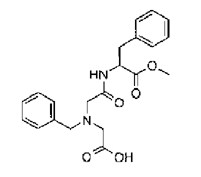

**(S)-2-[2-(Benzyl-carboxymethyl-amino)-acetylamino]-3-phenyl-propionic acid methyl ester (28)** To a stirred solution of H-L-Phe-OMe hydrochloride (360 mg, 1.66 mmol) in DMF (16 mL) were added DIEA (825µL) and N-benzyliminodiacetic anhydride acid (340mg, 1.66 mmol) in DMF (3 mL). The mixture was stirred overnight at room temperature and the solvent was evaporated. The crude product was purified by preparative HPLC to yield compound as a white solid (40%). Purity 100%; ¹H NMR (DMSO-*d6*] δ ppm: 8.13 (d, *J*=8.10 Hz, 1H), 7.31-7.16 (m, 10H), 5.5 8 (m, 1H), 3.69 (d, *J*=13.2 Hz, 1H), 3.64 (d, *J*=13.2 Hz, 1H), 3.61 (s, 3H), 3.22 (s, 2H), 3.16 (s, 2H), 3.08 (dd, *J=*5.4 and 13.8 Hz, 1H), 2.96 (dd, *J*=8.7 and 13.8 Hz, 1H), LC t_{R}= 4.39 min, MS (ESI+): m/z= 385(M+H)⁺.

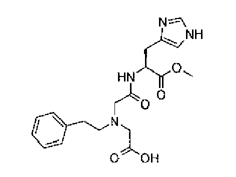

**(S)-2-[2-(Carboxymethyl-phenethyl-amino)-acetylamino]-3-(1*H*-imidazol-4-yl)-propionic acid methyl ester (29) as the formiate salt** was obtained from **(2)** and H-L-His-OMe as a white powder (52%). Purity 96%; ¹H NMR (DMSO-*d6* δ ppm:8.22 (d, *J*=7.8Hz, 1H), 8.14 (s, 1H), 7.56 (d, *J=*l.2Hz, 1H), 7.28-7.22 (m, 2H), 7.18-7.13 (m, 3H), 6.82 (d, *J*=1.2Hz, 1H), 4.52 (m, 1H), 3.58 (s, 3H), 3.38 (s, 2H), 3.25 (s, 2H), 2.92 (m, 2H), 2.76 (m, 2H), 2.66 (m, 2H); LC t_{R}= 2.63 min, MS (ESI+): m/z= 389(M+H)⁺.

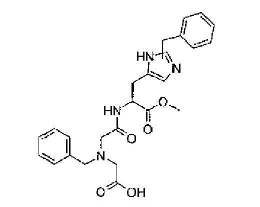

### (S)-2-[2-(Benzyl-carboxymethyl-amino)-acetylamino]-3-(3-benzyl-3H-imidazol-4-yl)-propionic acid methyl ester and (S)-2-[2-(Benzyl-carboxymethyl-amino)-acetylamino]-3-(1-benzyl-1H-imidazol-4-yl)-propionic acid methyl ester

**(30) as the formiate salt** was obtained from N-benzyl-iminodiacetic acid and H-L-His(Bzl)-OMe as a colorless oil (40%). Purity 98%; ¹H NMR (DMSO-*d6*)δ ppm: 8.40 (d, *J=*8.1 Hz, 1H), 7.68 (d, *J=*1.2 Hz, 1H), 7.3 6-7.15 (m, 10H), 6.90 (d, *J*=1.2Hz, 1H), 5.1 (s, 2H), 4.57 (m, 1H), 3.78 (d, *J=*13.2Hz, 1H), 3.72 (d, *J=*13.2 Hz, 1H), 3.52 (s, 3H), 3.24 (s, 2H), 2.92 (dd, *J*=6.9 and 15.0 Hz, 1H), 2.85 (dd, *J*=5.4 and 15.0 Hz, 1H). LC t_{R}= 3.36 min, MS (ESI+): m/z= 465 (M+H)⁺.

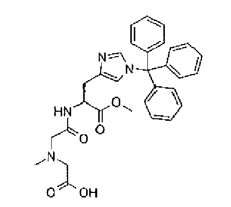

**(S)-2-[2-(Carboxymethyl-methyl-amino)-acetylamino]-3-(1-trityl-1*H*-imidazol-4-yl)-propionic acid methyl ester (31) as the formiate salt** was obtained as a white powder (33%) N-methyl-iminodiacetic acid and H-L-His-OMe Purity 98%; ¹H NMR (DMSO-*d6* δ ppm: 7.43 (d, *J=*1.5 Hz, 1H), 7.42-7.37 (m, 9 H), 7.16-7.11 (m, 6H), 6.76 (d, *J=*1.5 Hz, 1H), 4.73 (m, 1H), 3.65 (s, 3H), 3.61 (s, 2H), 3.48 (s, 2H), 3.09 (dd, *J*=5.4 and 15.0 Hz, 1H), 2.98 (dd, *J*=7.8 and 14.7 Hz, 1H), 2.64 (s, 3H). LC t_{R}= 4.21 min, MS (ESI+): m/z= 541 (M+H)⁺.

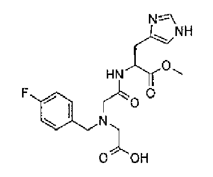

**(S)-2-{2-[Carboxymethyl-(4-fluoro-benzyl)-amnio]-acetylamino}-3-(1H-imidazol-4-yl)-propionic acid methyl ester (32) as the formiate salt** from **(1)** and H-L-His-OMe (20%) Purity 100%; ¹H NMR (DMSO-*d6*) δ ppm: 8.38 (d, *J=*8.1 Hz, 1H), 7.58 (d, *J*=0.9 Hz, 1H), 7.35-7.30 (m, 2H), 7.15-7.09 (m, 2H), 6.84 (d, *J=*0.9 Hz, 1H), 4.57 (m, 1H), 3.75 (d, *J=*13.2 Hz, 1H), 3.69 (d, *J=*13.2 Hz, 1H), 3.5 (s, 3H), 3.23 (s, 2H), 3.22 (s, 2H), 2.98 (dd, *J*=6.9 and 15 Hz, 1H), 2.92 (dd, *J*=5.4 and 14.7 Hz, 1H). LC t_{R}= 2.54 min, MS (ESI+): m/z= 393(M+H)⁺.

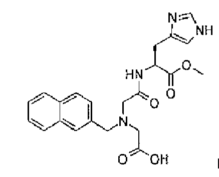

**(S)-2-[2-(Carboxymethyl-naphthalen-2-ylmethyl-amino)-acetylamino]-3-(1H-imidazol-4-yl)-propionic acid methyl ester (33) as the formiate salt** was obtained from **(5)** and H-L-His-OMe as a white powder (45%). Purity 100%; ¹H NMR (DMSO-*d*6) δ ppm: 2.99 (m, 2H), 3.29 (s, 2H), 3.32 (s, 2H), 3.59 (s, 3H), 3.90 (d, *J*=13.5Hz, 1H), 3.95 (d, *J=*13.5Hz, 1H), 4.59 (m, 1H) , 6.91 (d, *J*=0.9 Hz, 1H), 7.49-7.56 (m, 3H), 7.71 (d, *J=*0.9 Hz, 1H), 7.78 (s, 1H), 7.82-7.90 (m, 3H), 8.14 (s, 1H), 8.44 (d, *J*= 8.1 Hz, 1H); LC t_{R}= 3.46 min, MS (ESI+): m/z= 425(M+H)⁺.

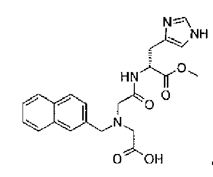

**(R)-2-[2-(Carboxymethyl-naphthalen-2-ylmethyl-amino)-acetylamino]-3-(1H-imidazol-4-yl)-propionic acid methyl ester (34) as the formiate salt** was obtained from **(5)** and H-D-His-OMe as a white powder (40%). Purity 100%; ¹H NMR (DMSO-*d6*) δ ppm: 2.99 (m, 2H), 3.29 (s, 2H), 3.32 (s, 2H) , 3.59 (s, 3H), 3.90 (d, *J=*13.5Hz, 1H), 3.95 (d, *J*=13.5Hz, 1H), 4.59 (m, 1H) , 6.89 (d, *J*=0.9 Hz, 1H), 7.49-7.56 (m, 3H), 7.67 (d, *J=*0.9 Hz, 1H), 7.78 (s, 1H), 7.82-7.90 (m, 3H), 8.14 (s, 1H), 8.44 (d, J= 8.1 Hz, 1H); LC t_{R}= 3.46 min, MS (ESI+): m/z= 425(M+H)⁺.

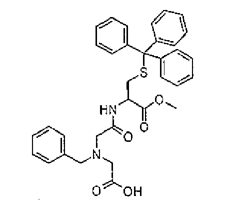

**(S)-2-[2-(Benzyl-carboxymethyl-amino)-acetylamino]-3-(tritylsulfanyl)-propionic acid methyl ester (35) as the formiate salt** was obtained from N-benzyl-iminodiacetic acid and **(17)** as a white powder (37%). Purity 98%; ¹H NMR (DMSO-*d6*) δ ppm : 2.46 (dd, J = 12Hz and = 4.8Hz, 1H), 2.61 (dd, J = 7.8Hz and 12.3Hz, 1H), 3.26 (s, 2H), 3.32 (s, 2H), 3.56 (s, 3H), 3.78 (s, 2H), 4.28 (dd, J = 5.1Hz and 7.8Hz, 1H), 7.26-7.35 (m, 15H+5H), 8.26 (d, J = 8.1Hz, 1H) ;¹³C NMR (DMSO-*d6*) δ ppm: 33.3, 51.2, 52.7, 54.2, 56.9, 57.8, 66.6, 127.3, 127.7, 128.5, 129.3, 129.4, 138.5, 144.5, 170.6, 170.9, 172.7. LC t_{r =} 6.93 min; MS (ESI+): m/z - 389 (M+H)⁺.

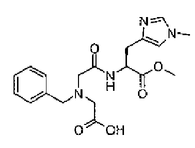

**(S)-2-[2-(Benzyl-carboxymethyl-amino)-acetylamino]-3-(1-methyl-1H-imidazol-4-yl)-propionic acid methyl ester (36) as the formiate salt** was obtained from N-benzyl-iminodiacetic acid and H-L-His(1-Me)-OMe as a white powder (19%). Purity 100%;¹H NMR (DMSO-*d6*) δ ppm : 2.88 (dd, *J=* 5.3 and 14.5 Hz, 1H), 2.95 (dd, *J=* 6.8 and 14.5 Hz, 1H), 3.25 (s, 4H), 3.53 (s, 3H), 3.59 (s, 3H), 3.73 (d, J = 13.2 Hz, 1H), 3.79 (d,*J=* 13.2 Hz, 1H), 4.57 (m, 1H), 6.87 (s, 1H), 7.25-7.32 (m, 5H), 7.55 (s, 1H), 8.36 (d,*J=*7.9 Hz, 1H). ¹³CNMR(DMSO-*d6) δ* ppm: 172.2, 171.7, 170.1, 138.2, 137.6, 136.2, 128.9, 128.3, 127.3, 118.2, 57.5, 56.8, 53.3, 51.9, 51.7, 32.9, 29.3. LC tᵣ = 2.35 min; MS (ESI+) : m/z - 389 (M+H)⁺.

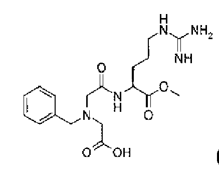

**(S)-2-[2-(Benzyl-carboxymethyl-amino)-acetylamino]-5-guanidino-pentanoic acid methyl ester (37) as the formiate salt** was obtained from H-L-Arg-OMe dihycrochloride and N-benzyl-iminodiacetic acid as a white powder (99 %) Purity 99% ;¹H NMR (DMSO-*d6*) δ ppm : 1.44-1.51 (m, 2H), 1.70-1.79 (m, 2H), 3.07-3.14 (m, 2H), 3.26 (s, 2H), 3.34 (s, 2H), 3.63 (s, 3H), 3.78 (s, 2H), 4.30 (ddd, *J*= 4.8Hz, 8.4Hz and 8.1Hz, 1H), 7.26-7.35 (m, 5H), 7.97 (m, 1H), 8.39 (d, J = 7.8Hz, 1H). ¹³C NMR (DMSO-*d6*) δ ppm: 24.9, 28.1, 41.5, 51.2, 51.9, 54.5, 56.4, 57.6, 127.2, 128.2, 128.8, 138.2, 157.0, 170.6, 172.2; 172.7. LC tᵣ = 2.51 min; MS (ESI+): m/z - 394 (M+H)⁺.

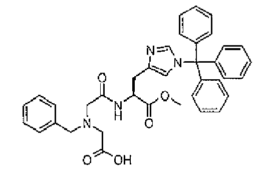

**(S)-2-[2-(Benzyl-carboxymethyl-amino)-acetylamino]-3-(1-trityl-1H-imidazol-4-yl)-propionic acid methyl ester (38) as the formiate salt** was obtained from N-benzyl-iminodiacetic acid and L-His(Trt)-OMe The crude product was purified by precipitation in H₂O as a white powder (80%) Purity 97%;¹H NMR (DMSO*-d6*) δ ppm: 2.91-2.94 (m, 2H), 3.24 (s, 2H), 3.26 (s, 2H), 3.52 (s, 3H), 3.77 (s, 2H), 4.58-4.65 (m, 1H), 6.66 (s, 1H), 7.01-7.04 (m, 6H), 7.15-7.18 (m, 5H), 7.33 (s, 1H), 7.35-7.38 (m, 9H), 8.47(d, J= 8.1Hz, 1H). ¹³C NMR (DMSO-*d6*) δ ppm: 29.5, 51.7, 51.9, 53.1, 56.9, 57.4, 74.7, 119.2, 127.2, 127.6, 127.8, 128.1, 128.2, 128.9, 129.3, 136.1, 137.9, 138.1, 142.1, 170.1, 171.6, 172.1. LC tᵣ = 5.08 min; MS (ESI+): m/z = 617 (M+H)⁺.

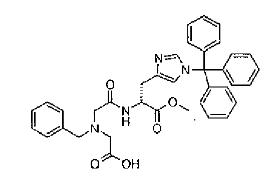

**(R)-2-[2-(Benzyl-carboxymethyl-amino)-acetylamino]-3-(1-trityl-1H-imidazol-4-yl)-propionic acid methyl ester (39) as the formiate salt** was obtained was obtained from N-benzyl-iminodiacetic acid and D-His(Trt)-OMe The crude product was purified by precipitation in H₂O to give a white powder (88%) Purity 97% ;¹H NMR (DMSO-*d6*) δ ppm: 2.91-2.94 (m, 2H), 3.24 (s, 2H), 3.26 (s, 2H), 3.52 (s, 3H), 3.77 (s, 2H), 4.58-4.65 (m, 1H), 6.66 (s, 1H), 7.01-7.04 (m, 6H), 7.15-7.18 (m, 5H), 7.33 (s, 1H), 7.35-7.38 (m, 9H), 8.47(d, J = 8.1Hz, 1H). ¹³C NMR (DMSO-*d6*) δ ppm: : 29.5, 51.7, 51.9, 53.1, 56.9, 57.4, 74.7,119.2, 127.2, 127.6, 127.8, 128.1, 128.2,128.9,129.3, 136.1, 137.9, 138.1, 142.1, 170.1, 171.6, 172.1. LC tᵣ = 5.08 min; MS (ESI+) : m/z = 617 (M+H)⁺.

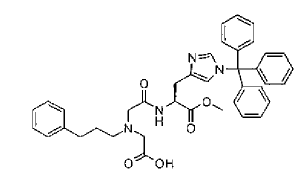

**(S)-2-{2-[Carboxymethyl-(3-phenyl-propyl)-amino]-acetylamino}-3-(1-trityl-1*H*-imidazol-4-yl)-propionic acid methyl ester (40) as the formiate salt** was obtained from **(4)** and L-His(Trt)-OMe as a white powder (57%). Purity 92% ;¹H NMR (DMSO-*d6*) δ pm: 1.62 (dt, J = 7.4Hz, 2H), 2.5 (m, 2H), 2.59 (t, J = 7.4Hz, 2H), 2:88 (m, 2H), 3.19 (s, 2H), 3.28 (s, 2H), 3.51 (s, 3H), 4.58 (ddd, J= 6Hz, 8.1Hz, 1H), 6.62 (s, 1H), 6.99-7.3 8 (m, 21 H), 8.49 (d, J = 8.2 Hz, 1H). ¹³C NMR (DMSO-*d6*) δ ppm: 29.3, 29.8, 32.7, 51.8, 51.8, 53.8, 55.1, 57.8, 119.0, 125.6, 127.5, 127.8, 128.0, 128.2, 129.2, 136.3, 137.6, 142.1, 170.6, 171.7, 172.7. LC tᵣ = 5.21 min; MS (EST+): m/z - 645 (M+H)⁺.

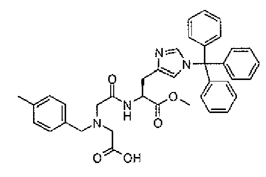

**(S)-2-{2-[Carboxymethyl-(4-methyl-benzyl)-amino]-acetylamino}-3-(1-trityl-1H-imidazol-4-yl)-propionic acid methyl ester (41) as the formiate salt** was obtained from **(3)** and L-His(Trt)-OMe as a white powder (37%). Purity 95%; ¹H NMR (DMSO-*d6*) δ ppm: 2.21 (s, 3H), 2.87 (dd, *J=* 5.0 and 14.5 Hz, 1H), 2.95 (dd, *J=* 6.2 and 14.5 Hz, 1H), 3.18 (s, 2H), 3.25 (s, 2H), 3.51 (s, 3H), 3.71 (s, 2H), 4.58-4.64 (m, 1H), 6.64 (d, *J* = 1.2Hz, 1H), 6.95 (d, J = 7.9Hz, 2H), 7.01-7.04 (m, 6H), 7.21 (d, J = 7.9Hz, 2H), 7.26 (d, *J=* 1.2Hz, 1H), 7.35-7.39 (m, 9H), 8.57 (d, *J* = 8.4Hz, 1H). ¹³C NMR *(DMSO-d6)* δ ppm: 20.7, 29.6, 51.8, 51.8, 53.2, 57.0, 57.1 , 74.5, 119.1, 128.2, 128.3, 128.7, 128.9, 129.2, 135.1, 136.2, 137.7, 142.1, 170.2, 171.6, 172.3. LC t_{r =} 5.28 min; MS (ESI+): m/z = 631 (M+H)⁺.

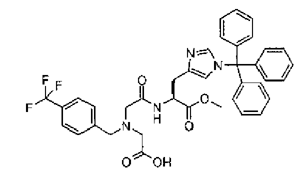

**(S)-2-{(2-[Carboxymethyl-(4-trifluoromethyl-benzyl)-amino]-acetylamino}-3-(1-trityl-1H-imidazol-4-yl)-propionic acid methyl ester (42) as the formiate salt** was obtained from **(6)** and L-His(Trt)-OMe (yield 75%). Purity 99%; ¹H NMR (DMSO-*d6)* δ ppm: 2.87 (dd, *J*=5.4 and 14.6 Hz, 1H), 2.95 (dd, *J=*6.5 and 14.6 Hz, 1H), 3.29 (s, 4H), 3.51 (s, 3H), 3.88 (s, 2H), 4.59 (m, 1H), 6.65 (d, *J=*1.2Hz*,* 1H), 7.00-7.04 (m, 6 H), 7.25 (d, *J*=1.2Hz,, 1H), 7.36 (m, 9 H), 7.5 (d, J = 8.1 Hz, 2H), 7.61 (d,J-8.1 Hz, 2H), 8.50 (d, *J=8.3* Hz, 1H). ¹³C NMR (DMSO-*d6*) δ ppm : 172.0, 171.6, 169.9, 143.3, 142.1, 137.9, 136.4, 129.9, 129.6, 128.6, 128.5, 126.41, 125.4, 119.2, 74.5, 56.9, 56.8, 53.3, 51.$, 29.5 LC tᵣ = 5.87 min ; MS (ESI+): m/z = 685 (M+H)⁺.

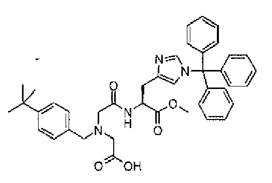

**(S)-2-{2-[(4-tert-Butyl-benzyl)-carboxymethyl-amino]-acetylamino}-3-(1-trityl-1H-imidazol-4-yl)-propionic acid methyl ester (43) as the formiate salt** was obtained from **(7)** and L-His(Trt)-OMe as a white powder (79%). Purity 94%; ¹H NMR (DMSO-*d6*) δ ppm: 8.47 (d, *J* = 8.1 Hz, 1H), 7.37 (m, 10H), 7.26 (d, *J*= 8.4 Hz, 2H), 7.20 (d, J= 8.4 Hz, 2H), 7.03 (m, 6H), 6.65 (s, 1H), 4.62 (m, 1H), 3.74 (s, 2H), 3.52 (s, 3H), 3.22 (s, 2H), 3.16 (s, 2H), 2.92 (m, 2H), 1.19 (s, 9H). ¹³C NMR (DMSO-*d6*) δ ppm: 172.1, 171.6, 170.1, 149.5, 142.1, 137.9, 136.3, 135.0, 129.2, 128.6, 128.2, 128.0, 124.9, 119.1, 74.5, 56.6, 56.5, 52.6, 34.1, 31.1, 29.9. LC tᵣ = 6.32 min; MS (ESI+): m/z = 673 (M+H)⁺.

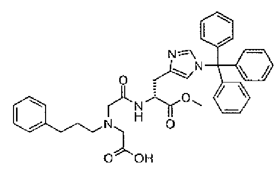

**(R)-2-{2-[Carboxymethyl-(3-phenyl-propyl)-amino]-acetylandino}-3-(1-trityl-1H-imidazot-4-y))-propionic acid methyl ester (44) as the formiate salt** was obtained from **(4)** and D-His(Trt)-OMe as a white powder (45%). Purity 99% ;¹H NMR (DMSO-*d6*) δ ppm: 1.62 (dt, J= 7.4Hz, 2H), 2.5 (m, 2H), 2.59 (t, J = 7.4Hz, 2H), 2.88 (m, 2H), 3.19 (s, 2H), 3.28 (s, 2H), 3.51 (s, 3H), 4.58 (dd, *J*= 6Hz, 8.1 Hz, 1H), 6.62 (s, 1H), 6.99-7.38 (m, 21 H), 8.49 (d, *J=* 8.2 Hz, 1H). ¹³C NMR (DMSO-*d6*) δ ppm: 29.3, 29.8, 32.7, 51.8, 51.8, 53.8, 55.1, 57.8, 119.0, 125.6, 127.5, 127.8, 128.0, 128.2, 129.2, 136.3, 137.6, 142.1, 170.6, 171.7, 172.7. LC tᵣ - 5.21 min; MS (ESI+) m/z = 645 (M+H)⁺.

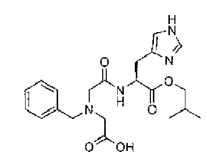

**(S)-2-[2-(Benzyl-carboxymethyl-amino)-acetylamino]-3-(1H-imidazol-4-yl)-propionic acid isobutyl ester (45) as the formiate salt** was obtained from **(18)** and N-benzyl-iminodiacetic acid as a white product (8%) Purity: 99 %; ¹H NMR (DMSO-*d6*) δ ppm: 8.44 (d, *J*=7.7 Hz, 1 H), 7.60 (s, 1H), 7.29-7.22 (m, 5H), 6.85 (s, 1H), 4.57 (m, 1H), 3.78 (m, 2H+2H), 3.26 (s, 2H), 3.24 (s, 2H), 3.00 (d, J = 6.7 and 14.7 Hz, 1H), 2.93 (dd, J= 5.4 and 14.7 Hz, 1H), 1.78 (m, 1 H), 0.79 (d, J =7.16 Hz, 6H). ¹³C NMR (DMSO-d6) δ ppm: 172.2, 171.3, 170.1, 138.1, 135.4, 134.0, 129.9, 128.3, 127.2, 115.6, 70.3, 57.5, 56.6, 53.4, 51.9, 28.9, 27.2, 18.7. LC: t_{R}= 3.52 min, MS: (ESI⁺): m/z= 417 (M+H)⁺.

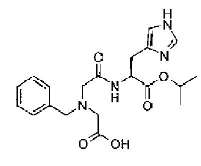

**(S)-2-[2-(Benzyl-carboxymethyl-amino)-acetylamino]-3-(1*H* imidazol-4-yl)-propionic acid isopropyl ester (46) as the formiate salt** was obtained from **(19)** and N-benzyl-iminodiacetic acid as a colorless oil (40%). Purity : 98%; ¹H NMR (DMSO-*d6*) δ ppm : 8.41 (d, *J=*8.10 Hz, 1H), 7.65 (s, 1H), 7.33-7.27 (m, 5H), 6.87 (s, 1H), 4.83 (sept, *J=*6.30 Hz, 1H), 4.51 (m, 1H), 3.80 (d, *J=*13.2Hz*,* 1H), 3.74 (d, *J=*13.2 Hz, 1H), 3.27 (s, 2H), 3.24 (s, 2H), 2.98 (dd, *J=*6.9Hz and 14.7 Hz, 1H), 2.92 (dd, J =5.4Hz and 14.7 Hz, 1H), 1.08 (d, J = 6.0 Hz, 6H); ¹³C NMR (DMSO-*d6*) δ ppm : 172.2, 170.6, 170.1, 138.1, 135.1, 133.5,128.9, 128.3, 127.2, 68.0, 57.4, 56.7, 53.3, 51.9, 28.7, 21.4; LC : tᵣ = 3.23min; MS (ESI+): m/z = 403 (M+H)⁺.

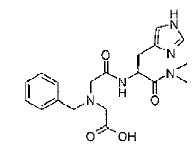

**(Benzyl-{[(S)-1-dimethylcarbamoyl-2-(1*H*-imidazol-4-yl)-ethylcarbamoyl]-methyl}-amino)-acetic acid (47) as the formiate salt** was obtained from **(20)** and N-benzyl-iminodiacetic acid as colourless oil (23%). Purity : 98% ; ¹H NMR (DMSO-*d6*) δ ppm : 8.17 (s, 2H), 8.12 (d, J= 8.4 Hz, 1H), 7.62 (s, 1H), 7.32-7.23 (m, 5H), 6.78 (s, 1H), 4.96 (m, 1H), 3.72 (s, 2H), 3.26 (s, 2H), 3.18 (s, 2H), 2.93 (s, 3H), 2.90-2.70 (m, 3H + 2H); ¹³C NMR (DMSO-*d6*) δ ppm : 172.3, 170.5, 169.5, 138.1, 134.7, 132.6, 128.9, 128.3, 127.2, 117.0, 57.6, 56.7, 54.0, 48.4, 36.4, 35.2, 29.5; LC : tᵣ = 2.6min; MS (ESI+) : m/z = 388 (M+H)⁺.

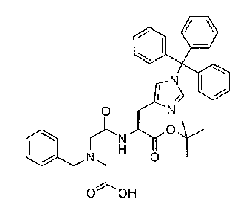

**(S)-2-[2-(Benzyl-carboxymethyl-amino)-acetylamino]-3-(1-trityl-1*H*-imidazol-4-yl)-propionic acid *tert-butyl* ester (48)** was obtained from N-benzyl-iminodiacetic acid and H-L-His(Trt)-OtBu as obtained as white powder (92 %) Purity : 99 %; LC : t_{R}= 5.55 min.

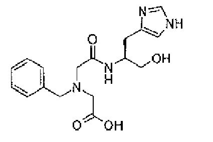

**(Benzyl- {[(S)-2-hydroxy-1-(1*H*-imidazol-4-ylmethyl)-ethylearbamoyl]-methyl)-amino)-acetic acid (49)** was obtained from N-benzyl-iminodiacetic acid and H-L-Histidinol as an oil (47 %). Purity : 97 %; ¹H NMR (DMSO-d6) δ ppm : 7.81 (d, *J* = 8.7 Hz, 1H), 7.71 (s, 1H), 7.27 (m, 5H), 6.83 (s, 1H), 3.98 (m, 1H), 3.71 (s, 2H), 3.37 (dd, J. 4.5 Hz and J- 10.6 Hz, 1H), 3.29 (dd, *J* = 6.0 Hz and *J*= 10.5 Hz, 1H), 3 .24 (s, 2H), 3.16 (s, 2H), 2.77 (dd, *J=* 6.0 Hz and J = 15.0 Hz, 1H), 2.67 (dd, J = 7.5 Hz and *J =* 15.0 Hz, 1H); ¹³C NMR (DMSO-*d6*) δ ppm : 172.5, 169.7, 138.2, 134.6, 133.4, 128.9, 128.4, 127.3, 117.0, 62.4, 57.7, 57.1, 54.3, 50.2, 28.0; LC : t_{R}= 2.24 min.

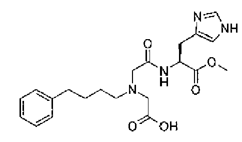

(S)-2-{2-[Carboxymethyl-(4-phenyl-butyl)-amino]-acetylamino}-**3-(1*H* imidazol-4-yl)-propionic acid methyl ester (50) as the formiate salt** was obtained from **(8)** and H-L-His-OMe as a white powder (73 %). Purity : 99 %; ¹H NMR (DMSO-d6) δ ppm : 8.28 (d, *J* = 8.6Hz, 1H), 8.04 (d, J = 1.2Hz, 1H), 7.28-7.12 (m, 5H), 7.01 (d, J = 1.2Hz, 1H), 4.59 (m, 1H), 3.59 (s, 1H), 3.30 (s, 2H), 3.19 (s, 2H), 3.02 (dd, J = 6.1Hz, J = 15.2Hz, 1H), 2.93 (dd, J = 7.3Hz, J = 15.2Hz, 1H), 2.56 (m, 4H), 1.52 (m, 2H), 1.40 (m, 1H);¹³C NMR (DMSO-d6) δ ppm : 172.5, 171.4, 170.6, 142.2, 134.7, 131.9, 128.3, 128.2, 125.6, 116.3, 57.6, 55.0, 54.0, 52.0, 51.5, 35.0, 28.5, 28.0, 26.7. LC: t_{R}= 3.41 min; MS : (ESI+): m/z= 417 (M+H)⁺.

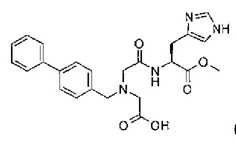

**(S)-2- [2-(Biphenyl-4-ylmethyl-carboxymethyl-amino)-acetylamino]-3-(1*H*-imidazol-4-yl)-propionic acid methyl ester (51) as the formiate salt** was obtained from **(10)** and H-L-His-OMe as an oil (61 %).Purity : 99 %; ¹H NMR (DMSO-d6) δ ppm : 8.15 (d, *J* = 8.1 Hz, 1H), 7.68 (d, *J* = 8.2 Hz, 2H), 7.61 (d, *J* = 8.2 Hz, 2H), 7.66 (s, 1H), 7.48-7.33 (m, 5H), 6.89 (s, 1H), 4.60 (m, 1H), 3.83 (d, J = 13.3 Hz, 1H), 3.76 (d, J= 13.3 Hz, 1H), 3.59 (s, 3H), 3.30 (s, 2H), 3.28 (s, 2H), 3.00 (m, 2H); ¹³C NMR (DMSO-d6) δ ppm: 172.2, 171.7, 170.2, 139.9, 139.1, 137.4, 135.2, 133.5, 129.4, 128.9, 127.3, 126.6, 115.8, 57.1, 56.8, 53.5, 53.4, 51.9, 28.7; LC : t_{R}= 3.73 min; MS (ESI+): mlz= 451 (M+H)⁺.

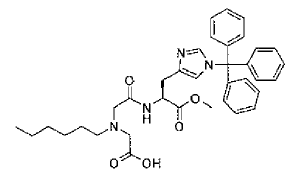

**(S)-2-[2-(Carboxymethyl-hexyl-amino)-acetylamino]-3-(1-trityl-1*H*-imidazol-4-yl)-propionic acid methyl ester (52) as the formiate salt** was obtained from **(9)** and H-L-His(Trt)-OMe as an oil (52 %). Purity : 83 %;LC : t_{R}= 4.93 min MS : (ESI+): m/z= 611 (M+H)⁺

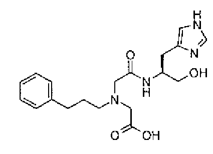

**[{[(S)-2-Hydroxy-1-(1*H*-imidazol-4-ylmethyl)-ethylcarbamoyl]-methyl}-(3-phenyl-propyl)-amino]-acetic acid (53) as the formiate salt** was obtained from (4) and H-L-Histidinol as colourless oil (30%). Purity : 98% ; ¹H NMR (DMSO-*d6*) δ ppm : 8.31 (s, 2H), 8.07 (d, *J* = 8.5 Hz, 1H), 7.50 (d, *J* = 1.2Hz, 1H), 7.28-7.15 (m, 5H), 6.76 (d, *J*= 1.2Hz, 1H), 3.92 (m, 1H), 3.35 (dd, *J*= 4.8 Hz and *J* = 10.6 Hz, 1H), 3.28 (dd, *J* = 6.1 Hz and J = 10.6 Hz, 1H), 3.19 (s, 2H), 3.10 (s, 2H), 2.75 (dd, *J* = 5.7 Hz and *J* = 14.6 Hz, 1H), 2.63 (dd, J = 7.3 Hz and J = 14.6 Hz, 1H), 2.5 (m, 2H+2H), 1.62 (qt, J = 8.1Hz,2H); ¹³C NMR (DMSO-*d6*) δ ppm : 173.2, 170.3, 164.5, 142.1, 134.6, 133.4, 128.3, 128.2, 125.6, 117.8, 62.4, 58.5, 56.6, 54.4, 50.4, 32.7, 29.1, 28.2; LC : tᵣ = 3.08min; MS (ESI+): m/z = 375 (M+H)⁺.

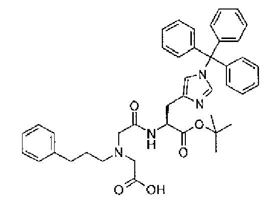

**(S)-2-{2-[Carboxymethyl-(3-phenyl-propyl)-amino]-acetylamino}-3-(1-trityl-1*H*-imidazol-4-yl)-propionic acid *tert-*butyl ester (54) as the formiate salt** from **(4)** and H-L-His-OtBu (48%). Purity : 97% ; ¹H NMR (DMSO-*d6*) δ ppm : 8.72 (d, J= 8.1Hz, 1H), 8.40 (s, 4H), 7.36-6.99 (m, 15H+5H+1H), 6.65 (s, 1H), 4.42 (m, 1H), 3.16 (s, 2H), 3.12 (s, 2H), 2.85 (m, 2H), 2.60-2.54 (m, 2H+2H), 1.60 (qt, J = 7.5Hz, 2H), 1.29 (s, 9H); LC : tᵣ = 6.02min; MS (ESI+) : m/z = 686 (M+H)⁺.

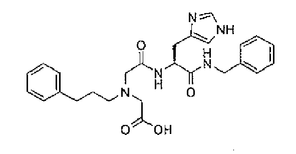

**[{[(S)-1-Benzylcarbamoyl-2-(1H-imidazol-4-yl)-ethylcarbamoyl]-methyl)-(3-phenyl-propyl)-amino]-acetic acid (55) as the formiate salt** was obtained from **(4)** and **(21)** (84%). Purity : 98% ; ¹H NMR (DMSO-*d6*) δ ppm : 8.38 (t, J = 5.5Hz, 1H), 8.30 (d, *J* = 8.5Hz, 1H), 8.28 (s, 1H), 7.5 (s, 1H), 7.11-7.29 (m, 10H), 6.76 (s, 1H), 4.55 (m, 1H), 4.24 (d, *J* = 5.5Hz, 2H), 3.27 (s, 2H), 3.21 (d, *J* = 16.6Hz, 1H), 3.14 (d, *J* = 16.6Hz, 1H), 2.94 (dd, *J* = 5.5Hz and *J* = 15Hz, 1H), 2.87 (dd, *J* = 7.4Hz and *J* = 15Hz, 1H), 2.52 (m, 4H), 1.64 (dt, *J* = 7.5Hz, 2H); ¹³C NMR (DMSO-*d6*) δ ppm : 173.0, 170.9, 170.6, 164.2, 142.1, 139.3, 134.7, 133.4, 128.3, 128.25, 128.2, 126.8, 126.6, 125.7, 125.6, 58.1, 55.9, 54.3, 52.9, 41.9, 32.7, 29.8, 29.7; LC : tᵣ = 5.02 min; MS (ES1+): m/z - 478 (M+H)⁺.

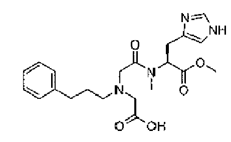

**(S)-2-({2-[Carboxymethyl-(3-phenyl-propyl)-amino]-acetyl)-methyl-amino)-3-(1*H*-imidazol-4-yl)-propionic acid methyl ester (56) as the formiate salt** was obtained from **(4)** and Me-L-His-OMe (85%). Purity : 98% ; ¹H NMR (DMSO-*d6*) δ ppm: 8.24 (s, 2H), 7.53 (s, 0.45H), 7.52 (s, 0.55H), 7.29-7.12 (m, 5H), 6.86 (s, 0.45H), 6.78 (s, 0.55H), 5.32 (dd, *J*= 5.1Hz and *J =* 10.2Hz, 0.45H), 4.99 (dd, J= 5.1Hz and J = 10.2Hz, 0.55H), 3.66 (s, 1.35H), 3.61 (s, 1.65H), 3.48 (s, 2H), 3.39 (s, 2H), 3.23-3.05 (m, 2H), 2.94 (s, 1.35H), 2.90(s, 2H), 2.72 (m, 0.9H+0.9H), 2.53 (m, 1.1H+1.1H), 1.63 (m, 2H); ¹³C NMR (DMSO-*d6*) δ ppm : 172.1, 171.0, 170.5, 164.0, 142.1, 134.9, 134.8, 133.8, 128.4, 125.9, 116.7, 116.2, 58.9, 58.4, 58.1, 56.7, 52.3, 52.0, 32.8, 32.7, 32.5, 28.1, 28.0, 25.6; LC : tᵣ= 3.58 8 min; MS (ES1+) : m/z = 417 (M+H)⁺.

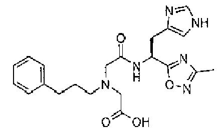

[**{[(S)-2-(1H-Imidazol-4-yl)-1-(3-methyl-[1,2,4] oxadiazol-5-yl)-ethylcarbamoyl]-methyl)-(3-phenyl-propyl)-amino]-acetic acid (57) as the formiate salt** was obtained from **(4)** and **(22)** (50%). Purity : 98% ; ¹H NMR (DMSO-*d6*) δ ppm : 8.97 (t, J = 7.8Hz, 1H), 8.27 (s, 1H), 7.5 (s, 1H), 7.12-7.28 (m, 5H), 6.79 (s, 1H), 4.23 (m, 1H), 3.28 (s, 2H), 3.22 (s, 2H), 2.94 (m, 2H), 2.69-2.50 (m, 4H), 2.26 (s, 3H), 1.64 (m, 2H); ¹³C NMR (DMSO-*d6*) δ ppm : 178.8, 173.1, 170.9, 166.1, 164.1, 142.0, 135.0, 132.9, 128.2, 125.6, 119.1, 58.9, 57.9, 46.3, 32.7 , 32.6, 30.3, 39.1, 11.0; LC : tᵣ = 3.68 min; MS (ESI+): m/z = 427 (M+H)⁺.

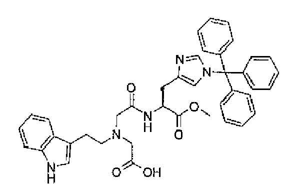

**(S)-2-(2-{Carboxymethyl-[2-(1H-indol-3-yl)-ethyl]-amino}-acetytamino)-3-(1-trityl-1H-imidazol-4-yl)-propionic acid methyl ester (58) as the formiate salt** was obtained from (11) and L-His(Trt)-OMe (45%). Purity : 98% ; LC : tᵣ = 6.93 min; MS (ESI+) : m/z = 670 (M+H)⁺.

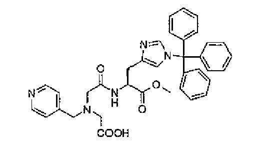

**(S)-2-[2-(Carboxymethyl-pyridin-4-ylmethyl-amino)-acetylamino]-3-(1-trityl-1H-imidazol-4-yl)-propionic acid methyl ester (59)** To a stirred solution of **(12)** (275mg, 1.23mmol) in THF (10mL) were added DCC (253mg, 1.23mmol). The resulting mixture was stirred at room temperature for 5 hours. Then L-His(Trt)-OMe (537mg, 1.19mmol) and DIEA (700µL, 3.84mmol) were added and the solution was stirred overnight. The insoluble were filtrated. The solvent was evaporated and the crude product was purified by preparative HPLC to give the compound as oil (309mg, 41%) Purity : 95% ; ¹H NMR (DMSO-*d6*) δ ppm : 8.65 (d, J = 4.8 Hz, 0.3H), 8.62 (d J = 4.8 Hz, 0.6H), 8.48 (d, J = 5.7 Hz, 1H), 8.36(d, J = 5.7 Hz, 1H), 8.2 (s, HCOOH), 7.36-7.18 (m, 15H+1H), 7.04-7.01 (m, 2H), 6.85 (s, 0.3H), 6.65 (s, 0.6H), 4.56 (m, 1H), 3.81 (s, 2H), 3.55 (s, 3H), 3.27 (s, 2H), 3.24 (s, 2H), 2.95 (m, 2H); LC : tᵣ = 4.59min; MS : (ESI+) : m/z = 618 (M+H)⁺.

### Example 5: synthesis of final compounds according tho the invention (60-61)

### Amide formation by reaction of activated carboxylic acid with an amine (60-61)

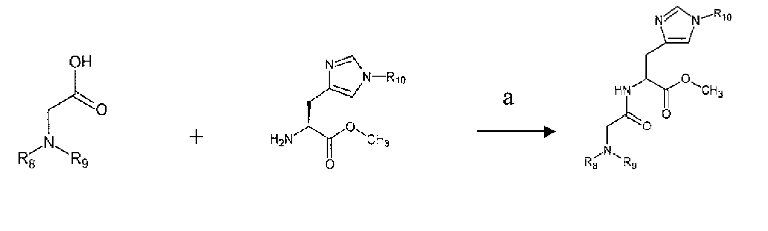

**Scheme 5:** *Reactants and conditions:* a) EDCI,HOBt,DIEA,DCM, rt, 12h

To a solution of the acid (0.5 mmol) in CH₂Cl₂ (10 mL) were added EDCI (0.55 mmol), HOBt (0.55 mmol), DIEA (2.5 mmol) and the amine (145 mg, 0.6 mmol). The mixture was stirred overnight at room temperature. The mixture was washed 3 times with 5 % NaHCO₃ aqueous solution. Organic layer was dried over MgSO₄ and evaporated. The crude product was purified by preparative HPLC.

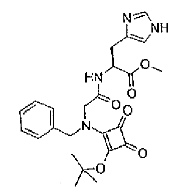

**(S)-2-{2-[Benzyl-(2-hydroxy-3,4-dioxo-cyclobut-1-enyl)-amino]-acetylamino}-3-(1H-imidazol-4-yl)-propionic acid methyl ester (60) as the formiate salt** was obtained from **(16)** and H-L-His-OMe as a white powder (37%). Purity 100%; LC t_{R}=4.3 min, MS (ESI+): m/z=469 (M+H)⁺.

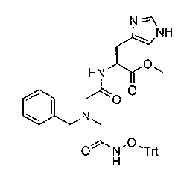

**(S)-2-[2-(Benzyl-hydroxycarbamoylmethyl-amino)-acetylamino]-3-(1H-imidazol-4-y1)-propionic acid methyl ester (61) as the formiate salt** was obtained from **(15)** and L-His-OMe as a white powder (51%). Purity 100%; LC t_{R}=59 min, MS (ESI+): m/z=633 (M+H)⁺.

### Exemple 6: synthsesis of protected compounds (62-66)

### Aminolysis of methyl esters (compounds 62-66)

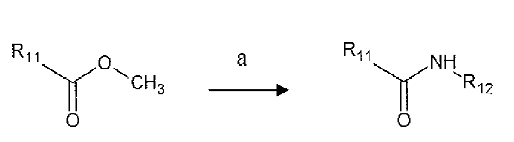

**Scheme 6 :** *Reactants and conditions :* a) CH₃-NH₂ , MeOH, rt or reflux or NH₃g, MeOH rt.

A stirred solution of ester (0.14mmol) in methanol (2 mL) was saturated with gaseous ammonia. The mixture was stirred for 24 hours with frequent saturation of the solution. The solvent was evaporated and the crude product was purified by preparative HPLC.

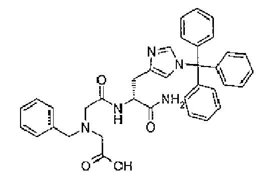

**(Benzyl-{[(R)-1-carbamoyl-2-(1-trityl-1H-imidazol-4-yl)-ethylcarbamoyl]-methyl}-amino)-acetic acid (62)** was obtained from **(39)** as a yellow oil and used without further purification (yield 100%).

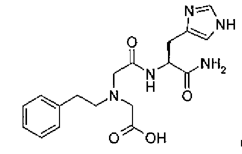

**({[(S)-1-Carbamoyl-2-(1H-imidazol-4-yl)-ethylcarbamoyl]-methyl}-phenethyl-amino)-acetic acid (63)** was obtained from **(29)** as a yellow oil (65%). Purity: 99 %; ¹H NMR (DMSO-d6) δ ppm: 8.07 (d, J=8.2 Hz, 1H), 7.62 (d, *J*=1.1Hz, 1H), 7.38 (s, 1H), 7.28-7.14 (m, 5H), 7.11 (s, 1H), 6.80 (d, J=1.0 Hz, 1H), 4.45 (m, 1H), 3.41 (d, J = 17.7 Hz, 1H), 3.33 (d, J = 17.7 Hz, 1H), 3.26 (d, J=16.8 Hz, 1H), 3.18 (d, J = 16.8 Hz, 1H), 2.94 (dd, J= 5.10 and 14.9 Hz, 1H), 2.85-2.63 (m, 5H) ¹³C NMR (DMSO-*d6*) δ ppm : 172.9, 172.7, 170.5, 139.9, 134.6, 133.2, 128.7, 128.3, 126.0, 116.6, 57.8, 56.5, 55.3, 52.1, 33.7, 29.5. LC: t_{R}= 2.25 min, MS: (ESI⁺): m/z= 374 (M+H)⁺.

To a stirred solution of ester (0.5mmol) in methanol (1 mL) were added 622 µL of a solution of methylamine in ethanol (33%). The mixture was refluxed overnight. The solvent was evaporated and the crude product was precipitated in water and filtrated to give the compound.

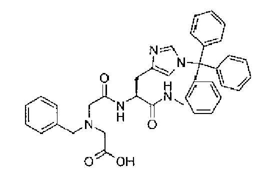

**(Benzyl-{[(S)-1-methylcarbamoyl-2-(1-trityl-1H-imidazol-4-yl)-ethylcarbamoyl]-methyl}-amino)-acetic acid (64) as the formiate salt** was obtained from **(38)** (100%). Purity : 97% ; LC : tᵣ = 4.5min; MS (ESI+) : m/z = 616 (M+H)⁺.

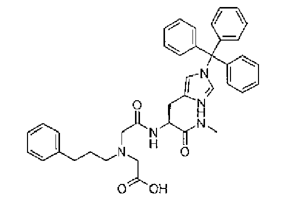

**[{[(S)-1-Methylcarbamoyl-2-(1-trityl-1H-imidazol-4-yl)-ethylcarbamoyl]-methyl}-(3-phenyl-propyl)-amino]-acetic acid (65)** was obtained from **(40)** (49%). Purity : 97% ; LC : tᵣ = 5.27min; MS (ESI+) : m/z = 644 (M+H)⁺.

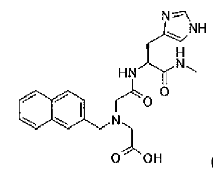

**({[(S)-2-(1H-Imidazol-4-yl)-1-methylcarbamoyl-ethylcarbamoyl]-methyl}-naphthalen-2-ylmethyl-amino)-acetic acid (66)** was obtained from **(33)** (100%).Purity : 97% ; ¹H NMR (DMSO-d6) δ ppm: 8.90 (d, *J* = 8.3Hz, 1H), 8.05 (q, J = 4.9Hz, 1H), 7.89-7.8 (m, 4H), 7.53-7.44 (m, 3H+1H), 6.76 (s, 1H), 4.41 (m, 1H), 3.84 (s, 2H), 3.16 (s, 2H), 3.05 (s, 2H), 2.91 (m, 2H), 2.59 (d, J = 4.9Hz, 3H); LC : tᵣ = 3.12min; MS (ESI+) : m/z = 424 (M+H)⁺.

### Exemple 7: synthesis of final products according to the invention (67-82)

### General procedure for deprotection (67-82):

The tritylated compound or t-Bu-squaric derivative was deprotected in DCM with TFA (5-50% volume). Triisopropylsilane was added until the yellow solution became colorless. The mixture was stirred at room temperature for 1H. The solvent was evaporated and the crude product was triturated in diethyl ether and purified by preparative HPLC if necessary.

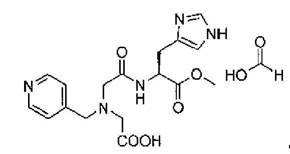

**(S)-2-[2-(Carboxymethyl-pyridin-4-ylmethyl-amino)-acetylamino]-3-(1H-imidazol-4-yl)-propionic acid methyl ester (67)** as formiate salt was obtained from **(59)** purity : 99% ; ¹H NMR (DMSO-*d6*) δ ppm : 8.5-8.47 (m, 1H+2H), 8.2 (s, HCOOH), 7.59 (d, J = 1.2 Hz, 1H), 7.33 (d, J = 5.7 Hz, 2H), 6.85 (d, J = 1.2Hz, 1H), 4.55 (m, 1H), 3.80 (s, 2H), 3.58 (s, 3H), 3.27 (s, 2H), 3.24 (s, 2H), 2.96 (m, 2H); ¹³C NMR (DMSO-*d6*) δ ppm: 172.5, 171.9, 170.3, 163.8, 149.6, 147.8, 135.6, 123.8, 116.8, 57.0, 56.6, 54.3, 52.1, 52.0, 28.8; LC (5min): tᵣ = 0.79min; MS : (ESI+): m/z = 376 (M+H)⁺.

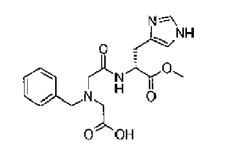

**(R)-2-[2-(Benzyl-carboxymethyl-amino)-acetylamino]-3-(1H-imidazol-4-yl)-propionic acid methyl ester .2CH₃COOH (68)** was obtained from **(39)** as a white powder (100%). Purity 99% ;¹H NMR (DMSO-*d6*) δ ppm: 3.16 (m, 2H), 3.32 (s, 2H), 3.39 (s, 2H), 3.65 (s, 3H), 3.81 (s, 1H), 4.70 (m, 1H), , 7.32 (m, 5H), 7.36 (s, 1H), 8.46 (d, *J=* 8.1Hz, 1H), 8.97 (s, 1H). ¹³C NMR (DMSO-*d6*) δ ppm: 26.1, 50.8, 52.3, 53.5, 55.9, 57.5, 117.0, 127.6, 128.3, 129.0, 129.2, 133.8, 136.9, 169.8, 170.8, 171.7. LC tᵣ = 0.783 min; MS (ESI+) : m/z = 375 (M+H)⁺.

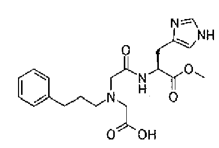

**(S)-2-{2-[Carboxymethyl-(3-phenyl-propyl)-amino]-acetylamino}-3-(1H-imidazol-4-yl)-propionic acid methyl ester.2CH₃COOH (69)** was obtained from **(40)** as a white powder (55%). Purity 99% ;¹H NMR (DMSO-*d6*) δ ppm: 1.79 (m, 2H), 2.57 (m, 2H), 2.90 (m, 2H), 3.11 (m, 2H), 3.62 (s, 3H), 3.70 (s, 2H), 3.78 (s, 2H), 4.70 (m, 1H), 7.17-7.30 (m, 5H), 7.41 (s, 1H), 8.86 (d, J = 7.2Hz, H), 8.98 (s, 1H). ¹³C NMR (DMSO-*d6*) δ ppm: 26.1, 26.8, 32.2, 51.2, 52.4, 54.7, 54.8, 55.7, 117.2, 126.0, 128.3, 128.4, 128.9, 133.7, 141.1, 167.5, 169.7, 170.6. LC tᵣ = 3.06 min; MS (ESI+): m/z = 403 (M+H)⁺,

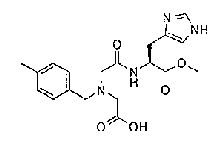

**(S)-2-{2-[Carboxymethyl-(4-methyl-benzyl)-amino]-acetylamino}-3-(1H-imidazol-4-yl)-propionic acid methyl ester.2CH₃COOH (70)** was obtained from **(41)** as a white powder (100 %). Purity 99%; ¹H NMR (DMSO-d6) δ ppm: 2.29 (s, 3H), 3.08 (dd, *J=* 9.0 and 15.3 Hz, 1H), 3.19 (dd, *J*=5.4 and 15.3 Hz, 1H), 3.41 (s, 2H), 3.45 (s, 2H), 3.65 (s, 3H), 3.83 (s, 2H), 4.70 (m, 1H), 7.15 (d, *J* = 8.1 Hz, 2H), 7.20 (d, *J* = 8.1 Hz, 2H), 7.38 (d, J = 1.2 Hz, 1H), 8.54 (d, *J* = 8.1 Hz, 1H), 8.98 (d, *J=* 1.2 Hz, 1H). ¹³C NMR (DMSO-*d6)* δ ppm: 171.0, 170.7, 169.7, 158.0, 137.1, 133.7, 132.7, 129.5, 129.1, 129.0, 117.1, 57.3, 55.5, 53.6, 52.4, 50.9, 26.2, 20.7. LC tᵣ = 2.86 min; MS (ESI+): m/z = 389 (M+H)⁺.

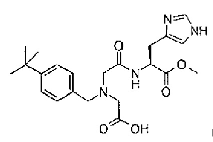

**(S)-2-{2-[(4-tert-Butyl-benzyl)-carboxymethyl-amino]-acetylamino}-3-(1H-imidazol-4-yl)-propionic acid methyl ester.2CH₃COOH (71)** was obtained from **(43)** as a colourless oil (84%). Purity 95%; ¹H NMR (DMSO-*d6*) δ ppm: 8.96 (s, 1H), 8.40 (d, *J* = 8.1 Hz, 1H), 7.36 (s, 1H), 7.33 (d, *J* = 8.4 Hz, 2H), 7.20 (d, *J* = 8.4 Hz, 2H), 4.70 (m, 1H), 3.71 (s, 2H), 3.65 (s, 3H), 3.30 (s, 2H), 3.26 (s, 2H), 3.19 (dd, J = 5.6 and 15.4 Hz, 1H), 3.09 (dd, J = 8.8 and 15.4 Hz, 1H),1.27 (s, 9 H) ¹³C NMR *(DMSO-d6)* δ ppm: 172.5, 171.3, 170.7, 150.2, 134.9, 134.2, 129.6, 129.3, 125.5, 117.6, 57.5, 56.7, 54.1, 52.8, 51.2, 34.7, 31.6, 26.7. LC tᵣ = 4.13 min; MS (ESI+) : m/z = 431 (M+H)⁺.

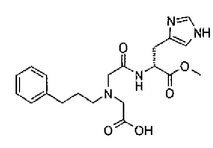

**(R)-2-{2-[Carboxymethyl-(3-phenyl-propyl)-amino]-acetylamino)-3-(1H-imidazol-4-yl)-propionic acid methyl ester .2CH₃COOH (72)** was obtained from **(44)** as a white powder (100%). Purity 94% ;¹H NMR *(DMSO-d6)* δ ppm: 1.79 (m, 2H), 2.57 (m, 2H), 2.90 (m, 2H), 3.11 (m, 2H), 3.62 (s, 3H), 3.70 (s, 2H), 3.78 (s, 2H), 4.70 (m, 1H), 7.17-7.30 (m, 5H), 7.41 (s, 1H), 8.86 (d, *J* = 7.2Hz, H), 8.98 (s, 1H). ¹³C NMR (DMSO-*d6*) δ ppm: 26.1, 26.8, 32.2, 51.2, 52.4, 54.7, 54.8 , 55.7, 117.2, 126.0, 128.3, 128.4, 128.9, 133.7, 141.1, 167.5, 169.7, 170.6. LC tᵣ = 3.06 min; MS (ESI+): m/z = 403 (M+H)⁺.

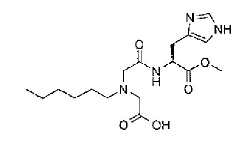

**(S)-2-[2-(Carboxymethyl-hexyl-amino)-acetylamino]-3-(1H-imidazol-4-yl)-propionic acid methyl ester (73) as the trifluoroacetate salt** was obtained from **(52)** as an oil (100%). Purity : 98% ; ¹H NMR (DMSO-*d6*) δ ppm : 8.99 (s, 1H), 8.80 (m, 1H), 7.41 (s, 1H), 4.70 (m, 1H), 3.76 (s, 2H), 3.65 (s, 5H), 3.19 (dd, *J=* 5.7 Hz and J - 15.3 Hz, 1H), 3 .07(dd, *J=* 9.3 Hz and *J=* 15.3 Hz, 1H), 2.85 (m, 2H), 1.45 (m, 2H), 1.22 (m, 6H), 0.85 (t, J= 6.3 Hz, 3H); ¹³C NMR (DMSO-*d6*) δ ppm : 171.1, 170.6, 168.5, 134.5, 129.0, 117.1, 56.1, 54.9, 54.7, 52.4, 51.1, 30.9, 26.5, 26.1, 25.9, 21.9, 13.9; LC : tᵣ = 3.03min; MS (ESI+): m/z - 369 (M+H)⁺,

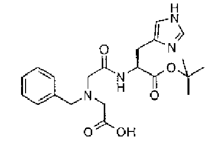

**(S)-2-[2-(Benzyl-carboxymethyl-amino)-acetylamino]-3-(1H-imidazol-4-yl)-propionic acid tert-butyl ester (74) as the formiate salt** was obtained from **(48)** as a colorless oil (41%). Purity 98% ; ¹H NMR (DMSO-*d6*) δ ppm : 8.38 (s, 1H), 8.32 (d, *J* = 8.1 Hz, 1H), 7.28 (m, 5H), 7.14 (s, 1H), 4.50 (m, 1H), 3.76 (s, 2H), 3.29 (s, 2H), 3.22 (s, 2H), 3.02 (m, 2H), 1.33 (s, 9H); ¹³C NMR (DMSO-*d6*) δ ppm : 172.3, 170.2, 169.9, 138.1, 134.7, 131.2, 128.9, 128.3, 127.3, 116.5, 81.1, 57.5, 56.5, 53.7, 51.8, 27.5, 27.4; LC : tᵣ = 3.41min; MS (ES1+) : m/z = 417 (M+H)⁺

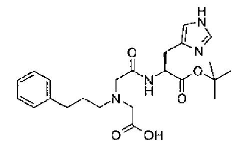

**(S)-2-{2-[Carboxymethyl-(3-phenyl-propyl)-amino]-acetylamino}-3-(1H-imidazol-4-yl)-propionic acid tert-butyl ester (75) as the formiate salt** was obtained from **(54)** as colourless oil (68%). Purity : 99% ; ¹H NMR (DMSO-*d6*) δ ppm : 8.54 (d, J = 8.6Hz, 1H), 8.32 (s, 2H), 7.48 (s, 1H), 7.27-7.14 (m, 5H), 6.81 (s, 1H), 4.41 (m, 1H), 3.24 (s, 2H), 3.16 (s, 2H), 2.88 (m, 2H), 2.60-2.54 (m, 2H+2H), 1.60 (qt, J= 7.5Hz, 2H), 1.29 (s, 9H); ¹³C NMR (DMSO-*d6*) δ ppm, 173.2, 170.7, 170.3, 164.6, 142.1, 134.9, 133.0, 128.3, 128.2, 125.6, 116.5, 80.5, 58.0, 56.1, 54.2, 52.5, 32.7, 29.3, 29.0, 27.6; LC : tᵣ = 4,19min; MS (ESI-) : m/z = 443 (M-H)-.

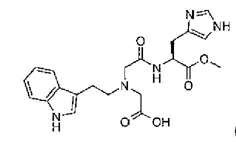

**(S)-2-(2-{Carboxymethyl-[2-(1H-indol-3-yl)-ethyl]-amino)-acetylamino)-3-(1H-imidazol-4-yl)-propionic acid methyl ester (76).2CF₃COOH** was obtained from **(58)** as a white powder (86%). Purity : 97% ; ¹H NMR (DMSO-*d6*) δ ppm : 10.92 (sl, NH), 8.97 (s, 1H), 8.93 (m, 1H), 7.53 (d, *J* = 7.8Hz, 1H), 7.40 (s, 1H), 7.35 (d, *J* = 8.1 Hz, 1H), 7.17 (d, J= 1.BHz, 1H), 7.08 (t, *J=* 7.2Hz, 1H), 6.98 (t, J = 7.8Hz, 1H), 4.71 (m, 1H), 3.96 (s, 2H), 3.87 (s, 2H), 3.62 (s, 3H), 3.22-2.98 (m, 6H); ¹³C NMR (DMSO-*d6*) δ ppm : 170.5, 169.4, 166.6, 136.3, 134.6,128.8, 126.8, 123.3, 121.3, 118.5, 118.2, 117.3, 111.6, 109.5, 55.7, 54.4, 54.9, 52.5, 51.4, 26.1, 21; LC : tᵣ = 4.17min; MS (ESI+) : m/z = 428 (M+H)⁺.

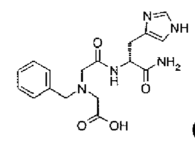

**(Benzyl-{[(R)-1-carbamoyl-2-(1H-imidazol-4-yl)-ethylcarbamoyl]-methyl)-amino)-acetic acid .2CF₃COOH** (77) was obtained from **(62)** as a white powder (100%). Purity 98 %; ¹H NMR (DMSO-*d6*) δ ppm: 8.95 (d, *J=* 1.4 Hz, 1H), 8.34 (d, *J* = 8.7 Hz, 1H), 7.54 (s, 2H), 7.32 (m, 5H), 7.29 (s, 1H), 4.57 (m, 1H), 3.88 (s, 2H), 3.47 (s, 2H), 3.45 (s, 2H), 3.15 (dd, J= 4.3 and 15.4 Hz, 1H), 2.96 (dd, J= 8.5 and 15.4 Hz, 1H). ¹³C NMR (DMSO-*d6*) δ ppm : 171.5, 158.6, 158.1, 135.4, 134.4, 129.8, 129.4, 128.5, 128.0, 116.7, 57.8, 55.8, 53.7, 51.1, 27.2. LC t_{R}= 1.98 min, MS (ESI+): m/z- 360 (M+H)⁺.

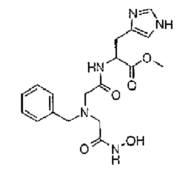

**(S)-2-[2-(Benzyl-hydroxycarbamoylmethyl-amino)-acetylamino]-3-(1H-imidazol-4-yl)-propionic acid methyl ester (78).2CF₃COOH was obtained from (61)** as a white powder (98%). Purity 100%; ¹H NMR (DMSO) δ ppm: 10.66 (s, 1H), 8.97 (d, *J*=1.2Hz, 1H), 8.68 (d, *J=* 8.1 Hz, 1H), 7.3 8-7.30 (m, 5H), 4.71 (m, 1H), 3.72 (s, 2H), 3.64 (s, 3H), 3.22-3.15 (m, 5H), 3.08 (dd, *J* = 9.2 and 15.3 Hz, 1H). LC t_{R}=2.6 min, MS (ESI+): m/z=390 (M+H)⁺.

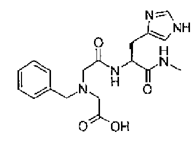

**(Benzyl-{[(S)-2-(1H-imidazol-4-yl)-1-methylcarbamoyl-ethylcarbamoyl]-methyl}-amino)-acetic acid (79). 2CF₃COOH** was obtained from **(64)** as colourless oil (70%).Purity : 98% ;¹H NMR (DMSO-*d6*) δ ppm : 8.95 (s, 1H), 8.39 (d, J = 8.1 Hz, 1H), 8.00 (q, J = 4.5 Hz, 1H), 7.33 (m, 5H), 7.28 (s, 1H), 4.57 (m, 1H), 3.91 (s, 2H), 3.51 (s, 2H), 3.49 (s, 2H), 3.14 (dd, *J* = 5.4 Hz and *J* = 15.3 Hz, 1H), 2.93 (dd, *J =* 8.1 Hz and *J* = 15.3 Hz, 1H), 2.60 (d, *J* = 4.5 Hz, 3H); ¹³C NMR (DMSO-*d6*) δ ppm : 170.8, 169.8, 168.5, 133.9, 135.3, 129.3, 129.8, 128.5, 128.1, 116.8, 57.9, 55.9, 53.7, 51.3, 27.3, 25.7; LC : tᵣ = 2.55min; MS (ESI+) : m/z = 374 (M+H)⁺.

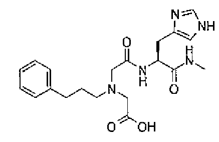

**[{[(S)-2-(1H-Imidazol-4-y1)-1-methylcarbamoyl-ethylcarbamoyl]-methyl}-(3-phenyl-propyl)-amino]-amino acid (80).2CF₃COOH** was obtained from **(65)** as colourless oil (58%). Purity : 98% ; ¹H NMR (DMSO-*d6*) δ ppm : 9.05 (d, *J* = 8.7Hz, 1H), 9.01 (s, 1H), 8.22 (q, *J* = 4.8Hz, 1H), 7.38 (s, 1H), 7.32-7.19 (m, 5H), 4.58 (m, 1H), 4.13 (m, 2H+1H), 4.08 (d, *J* = 6.6Hz, 1H), 3.17-3.13 (m, 2H+1H), 2.96 (dd, *J* = 8.7 Hz and *J =* 14.8 Hz, 1H),2.60-2.57 (m, 3H+2H), 1.92 (qt, J= 7.8Hz, 2H); ¹³C NMR (DMSO-*d6*) δ ppm : 169.5, 167.7, 165.2, 140.5, 134.1, 129.2, 128.4, 128.3,126.1,116.8, 55.3, 54.9, 54.1, 52.1, 31.8, 27.0, 25.7, 25.3; LC : tᵣ = 3.25min; MS (ESI+) : m/z - 402 (M+H)⁺.

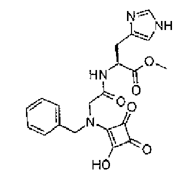

**(S)-2-{2-[Benzyl-(2-hydroxy-3,4-dioxo-cyclobut-1-enyl)-amino]-acetylamino}-3-(1H-imidazol-4-yl)-propionic acid methyl ester (81).CF₃COOH** was obtained from **(60)** as followed: **(60)** was dissolved in dichloromethane (2 mL) and cooled at 4°C in an ice/water bath. After 15 minutes of stirring, trifluoroacetic acid (2 mL) was added and the mixture was stirred at 4°C for 30 minutes then 10µL of distilled water were added. The reaction mixture was concentrated under reduced pressure and the crude product was precipitated in Et₂O and filtrated to give **(81)** (70 mg, 99%). Purity 100%; ¹H NMR (DMSO) δ ppm: 8.97 (d, *J*=1.5Hz, 1H), 8.57 (d, *J*=8.1Hz, 1H), 7.40-7.22 (m, 5H), 4.70-4.66 (m, 1H), 4.65 (d, *J*= 14.7Hz, 1H), 4.58 (d, *J*=14.7Hz, 1H), 4.00 (d, *J=* 16.5Hz, 1H), 3.95 (d, *J*=16.5Hz, 1H), 3.67 (s, 3H), 3.16 (dd, *J=* 5.1 and 15.0 Hz, 1H), 3.03 (dd, *J*=9.0 and 15.0 Hz, 1H). LC t_{R}=3.02 min, MS (ESI+): m/z=413 (M+H)⁺.

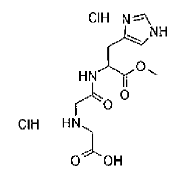

**(S)-2-[2-(Carboxymethyl-amino)-acetylamino]-3-(1H-imidazol-4-yl)-propionic acid methyl ester. 2HCl (82)** To a stirred solution of iminodiacetic acid (2 g, 15 mmol) in a dioxane/H₂O (3/1, 40mL) mixture were added, at 0°C, Boc₂O (3.93 g, 18mmol) and 10 mL of 2N NaOH solution. The mixture was stirred overnight at room temperature and dioxane was evaporated. The aqueous layer was acidified with a 20% citric acid solution and extracted by AcOEt. Organic layer was dried over MgSO₄ and evaporated to give the product as a white solid (2.36 g, 67%). To the diacid intermediate (1 g, 4.29 mmol) in solution in dry THF (20mL) was added DCC (0.88g, 4.29 mmol). The mixture was stirred overnight at room temperature and L-Hystidine methyl ester dihydrochloride (1.14 g, 4.67 mmol) and DIEA (2.1 mL) were added. After stirring at room temperature during 5 h, the precipitate was filtrated and the filtrate was evaporated. The crude product was purified by preparative HPLC to yield compound as a white solid (660 mg, 40%, 2 steps). Purity 99%; ¹H NMR (DMSO-*d6*) δ ppm: 8.95 (d, *J*=7.4Hz*,* 0.5H), 8.90 (d, *J*=7.4Hz*,* 0.5H), 7.74 (d, *J*=0.9Hz*,* 0.5H), 7.72 (d, *J*=0.9Hz, 0.5H), 6.88 (s, 1H), 4.50 (m, 1H), 3.85 (m, 2H), 3.83 (s, 2H), 3.58 (s, 3H), 2.95 (dd, *J*=5.7 and 14.9 Hz, 1H), 2.87 (dd, *J*=8.1 and 14.9Hz, 1H), 1.33 (s, 4.5H), 1.28 (4.5H), LC t_{R}=2.65 min, MS (ESI+): m/z=385 (M+H)⁺. Boc-protected derivative (300 mg, 0.78 mmol) was deprotected in presence of HCIg in DCM during 2h at room temperature. DCM was evaporated and the product was precipitated in Et₂O to give the compound as a white solid (214 mg, 77%). Purity 100%; ¹H NMR (DMSO-*d6*) δ ppm : 9.36 (d, *J*=7.5Hz*,* 1H), 9.09 (d, *J*=1..2Hz*,* 1H), 7.48 (s, 1H), 4.66 (m, 1H), 3.85 (s, 2H), 3.82 (s, 2H), 3.65 (s, 3H), 3.20 (dd, J =5.2 and 15.2Hz, 1H), 3.10 (dd, *J*=9.0 and 15.4 Hz, 1H); LC t_{R}=.0.68 min, MS (ESI+): m/z=285 (M+H)⁺.

### Example 8: synthesis of final compounds from amines and halides (85)

### Synthesis from amines and halides

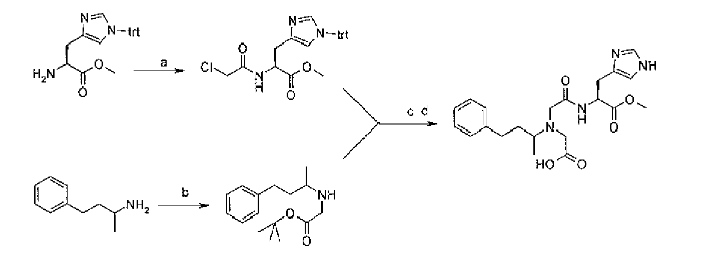

**Scheme 7:** *Reactants and conditions* a) chloroacetylchloride, NaHCO₃, DCM, room temp.,10min b) tert-butylbromoacetate, DIEA, THF, 0°C then room temp., overnight c)NaHCO₃ DMF, reflux, 24h d) TFA, TIS,DCM, room temp. 2h

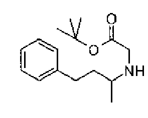

**(1-Methyl-3-phenyl-propylamino)-acetic acid tert-butyl ester (83).** To a stirred solution of 1-methyl-3-phenylpropylamine (321µL, 2mmol) and DIEA (870µL, 5mmol) in THF (5mL) were added dropwise at 0°C tert-butylbromoacetate (322µL, 2mmol). The resulting mixture was stirred at room temperature overnight. The insolubles were filtrated the organic layer was washed with brine, dried over MgSO4 and the solvent was evaporated. The crude product was purified by flash chromathography to give **(83)**(332mg, 63%). Purity : 97% ; ¹H NMR (DMSO-*d6*) δ ppm : 7.25-7.15 (m, 5H), 3.30 (m, 1H), 3.20 (s, 2H), 2.58 (m, 2H), 1.71-1.51 (m, 2H), 1.41 (s, 9H), 0.99 (d, J = 6.2 Hz, 2H); LC (10min): tᵣ = 4.73min; MS : (ESI+) : m/z = 264 (M+H)⁺.

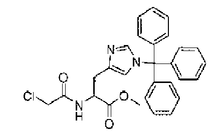

**(S)-2-(2-Chloro-acetylamino)-3-(1-trityl-1H-imidazol-4-yl)-propionic acid methyl ester (84):** To a stirred solution of L-His(Trt)-OMe (895mg, 2mmol) and NaHCO₃ (420mg, 5mmol) in DCM (10mL) were added dropwise chloroacetylchloride (159µL, 2mmol). The mixture was stirrd at room temperature 10minutes. The insoluble were eliminated and the solvent evaporated. (1.14g, quantitative yield).Purity : 98%; ¹H NMR (DMSO-*d6*) δ ppm : 8.63 (d, J = 7.8 Hz, 1H), 7.66 (s, 1H), 7.47-7.36 (m, 9H), 7.11-7.06 (m, 6H), 6.82 (s, 1H), 4.55 (m, 1H), 4.09 (s, 2H), 3.58 (s, 1H), 2.96 (dd, J= 5.7 Hz, J= 14.7 Hz, 1H), 2. 88 (dd, J= 8.3 Hz, J= 14.7 Hz, 1H); LC (5min): tᵣ = 2.81 min; MS : (ESI+) : m/z = 488 (M+H)⁺. 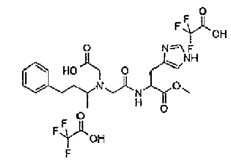 **(S)-2-{2-[Carboxymethyl-(1-methyl-3-phenyl-propyl)-amino]-acetylamino}-3-(1H-imidazol-4-yl)-propionic acid methyl ester (85).** To a stirred solution of **(84)** (102mg, 0.2mmol) and NaHCO₃ (66mg, 0.6mmol) in DMF anhydre (1mL) were added **(83)** (61mg, 0.23mmol). the mixture was stirred at room temperature 1h then was refluxed 20hours. KI (15mg, 0.09mmol) were added and the solution was refluxed one hour. The solvent was evaporated and the residue was solubilized in water, insoluble were filtrated. This solide was purified by preparative HPLC to give **(85)** as an oil (65mg, 45%). Purity : 98%; ¹H NMR (DMSO-*d6*) δ ppm : 8.43 (d, J = 8.4 Hz, 1H), 7.37-6.99 (m, 15H+5H+1H), 6.60 (s, 1H), 4.60 (m, 1H), 3.69-3.61 (m, 2H+2H+3H), 3.20-3.09 (m, 1H+2H), 2.61 (3, 2H), 1.80-1.46 (m, 2H), 0.97 (m, 3H); LC (5min): tᵣ = 3.94min; MS : (ESI+): m/z = 715 (M+H)⁺. Then protections were removed using TFA/DCM (50/50).Purity : 99% ; ¹H NMR (DMSO-*d6*) δ ppm : 8.98 (s, 1H), 8.92 (d, J = 8.1Hz, 1H), 7.4 (s, 1H), 7.37-7.17 (m, 5H), 4.72 (m, 1H), 3.69-3.61 (m, 2H+2H+3H), 3.20-3.01 (m, 1H+2H), 2.57 (t, J = 7.9 Hz, 2H), 1.85 (m, 1H), 1.59 (m, 1H), 1.13 (m, 3H); ¹³C NMR (DMSO-*d6*) δ ppm : 170.5, 158.6, 158.1, 141.4, 135.14, 128.7, 128.3, 128.2, 125.9, 117.2, 52.4, 51.2, 33.7, 31.8, 26.3, 26.4, 14.3; LC : tᵣ = 2.22min; MS : (ESI+): m/z = 417 (M+H)⁺.

### Example 9: synthesis of final compound (90, 91, 93-96, 101)

### Synthesis of tetrazoles

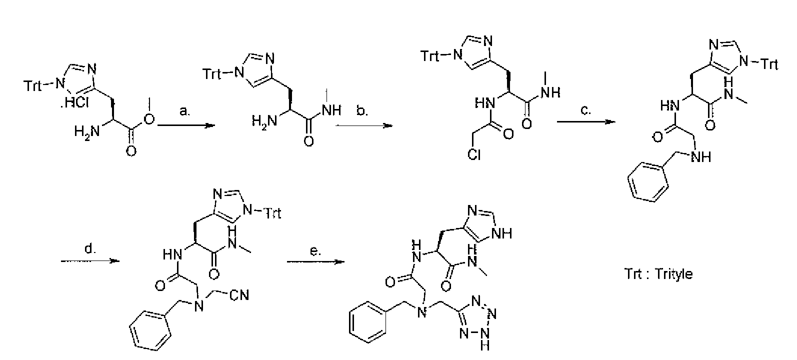

**Scheme 8:** *Reactants and conditions:* a) H₂N-CH₃ in EtOH 33 %wt, refluxed MeOH, overnight; b) DCM, NaHCO₃ AcCI room temp., 1 h. c) benzylamine, Na₂CO₃, KI, DMF, room temp. overnight then 60°C, 1.5 h. d) K₂CO₃, chloroacetonitrile, DMF, 60°C, 24 h. e) i. sodium azide, ammonium chloride, DMF, 90°C then ii. TFA 25% in DCM, trimethylsilane, room temp. 3 h.

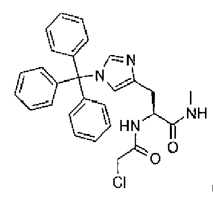

**(S)-2-(2-Chloro-acetylamino)-N-methyl-3-(1-trityl-1H-imidazol-4-yl)-propionamide (86)** To a stirred suspension of **(23)** (1 g, 2,43 mmol) in dichloromethane (25 mL), were added NaHCO₃ (409 mg, 4,87 mmol) and acetylchloride (213µL, 1,68 mmol) at room temperature. After an hour, the mixture was washed with water, saturated NaHCO₃ and brine. The organic layer was dried over MgSO₄ and the solvent was evaporated to give an yellow solid (1,2 g, quant.). Purity : 95% ; 1H NMR (DMSO-d6) δ (ppm): 8.35 (d, J = 8.3Hz, CONH), 7.91 (q, J = 4.7Hz, CONHMe), 7.40-7.37 (m, 10H), 7.23 (d, J =1.4Hz, 1H), 7.06-7.03 (m, 5H), 6.62 (d, J =1.2Hz, 1H), 4.41 (ddd, J = 8.4, 8.3 and 5.6 Hz, 1H), 4.04 (d, J = 13.5Hz, 1H), 3.98 (d, J = 13.5Hz, 1H), 2.86 (dd, J = 14.4Hz and 5.6Hz, 1H), 2.70 (dd, J = 14.4Hz and 8.4Hz, 1H), 2.52 (d, J = 4.7Hz, 3H); LC : tr = 3.10 min MS (ESI+): m/z = 487 (M+H)+

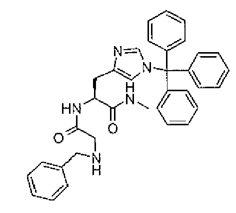

**(S)-2-(2-Benzylamino-acetylamino)-N-methyl-3-(1-trityl-1H-imidazol-4-yl)-propionamide (87):** To a stirred solution of benzylamine (270 µL, 2.4 mmol) in DMF were added **(86)** (800 mg, 1,6 mmol), Na₂CO₃ (348 mg, 3,2 mmol) and KI (82 mg, 0,49 mmol) at room temperature. After stirring overnight, the mixture was heated for 1h30 for completion of the reaction. The media was filtered, the solvent evaporated and the oil obtained was purified by silica gel chromatography (DCM/MeOH/Et₃N: 97/2/1) to give a white solid (712 mg, 78 %.). Purity : 90% ; 1H NMR (DMSO-d6) δ (ppm): 8.19 (d, J = 8.5Hz, CONH), 7.79 (q, J = 4.5Hz, CONHMe), 7.25-7.20 (m, 10H), 7.04-7.01 (m, 5H), 7.06-7.03 (m, 5H), 6.57 (d, J =1.2Hz, 1H), 4.46 (ddd, J = 8.2, 7.5Hz and 5.6 Hz, 1H), 3.6 (s, 2H), 3.08 (d, J = 16.3Hz, 1H), 3.01 (d, J = 16.3Hz, 1H), 2.86-2.77 (m, 2H), 2.51 (d, J = 4.5Hz, 3H) ; LC : tr = 2.95 min MS (ESI+): m/z = 558 (M+H)+.

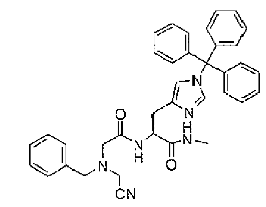

**(S)-2-[2-(Benzyl-cyanomethyl-amino)-acetylamino]-N-methyl-3-(1-trityl-1H-imidazol-4-yl)-propionamide (88):** To a stirred solution of **(87)** (662 mg, 1.18 mmol) in DMF (10 mL) were added K₂CO₃ (410 mg, 2.96 mmol) and chloroacetonitrile (190 µL, 2.3 mmol). The mixture was heated at 60°C for 24 hours. The media was filtered over celite and the oil obtained was purified by preparative HPLC (mass collect) to give a white solid (100 mg, 14%). Purity : 95% ; 1H NMR (CD3OD) δ (ppm) : 7.37-7.30 (m, 10H), 7.26-7.19 (m, 5H), 7.09-7.05 (m, 5H), 6.68 (d, J = 1.4Hz, 1H), 6.65 (d, J = 1.2Hz, 1H), 4.63 (dd, J = 7.5Hz and 5.3 Hz, 1H), 4.50 (d, J = 15.0Hz, 1H), 4.43 (d, J = 15.0Hz, 1H), 3.67 (s, 2H), 3.60 (d, J = 10.3Hz, 1H), 3.55 (d, J = 10.3Hz, 1H), 3.03 (dd, J = 14.8Hz and 5.3Hz, 1H), 2.92 (dd, J = 14.8Hz and 7.5Hz, 1H), 2.75 (s, 3H); LC : tr =3.42 min MS (ESI+): m/z = 597 (M+H)+.

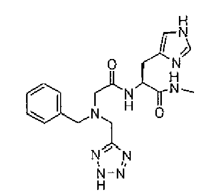

**(S)-2-{2-[Benzyl-(2H-tetrazol-5-ylmethyl)-amino]-acetylamino}-N-methyl-3-(1-trityl-1H-imidazol-4-yl)-propionamide (89):** To a stirred solution of **(88)** (64 mg, 0,1 mmol) in DMF were added sodium azide (42 mg, 0,64 mmol) and ammonium chloride (34 mg, 0,64). The mixture was heated at 90°C until completion of the reaction. The solvent was evaporated and the crude material was diluted in EtOH and filtered. The filtrate was concentrated under vacuo to give a brown oil which was diluted in dichloromethane (4 mL). Trifluoroacetic acid (25%) and trimethylsilane were added. The mixture was stirred at room temperature during 3 hours. The solvent was evaporated and washings with petroleum ether followed by evaporation allowed the elimination of the TIS. The crude material was purified by preparative HPLC to give an yellow solid (3 mg, 7%).Purity : 96% ; 1H NMR (CD3OD) δ (ppm): 8.07 (d, J = 1.1Hz, 1H), 7.32-7.25 (m, 6H), 6.95 (s, 1H), 4.63 (dd, J = 8.2Hz and 5.2Hz, 1H), 3.95 (d, J = 14.1Hz, 1H), 3.88 (d, J = 14.1Hz, 1H), 3.65 (s, 2H), 3.21-3.04 (m, 4H), 2.74 (s, 3H) ; 13C NMR (CD3OD) δ (ppm): 172.2, 171.8, 167.6, 158.4, 137.2, 134.4, 131.5, 129.1, 128.1, 127.2, 117.2, 58.6, 56.4, 52.5, 28.2, 25.1; LC : tr =1.95 min MS (ESI+) : m/z = 398 (M+H)+.

### Methyl ester derivatives 90-101.

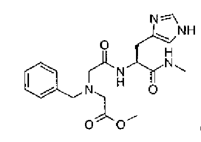

**(Benzyl-{[(S)-2-(1H-imidazol-4-yl)-1-methylcarbamoyl-ethylcarbamoyl]-methyl}-amino)-acetic acid methyl ester (90)** was obtained from (79) as followed. To a stirred solution of (70) (86mg, 0.14mmol) in methanol (4mL) at 0°C were added dropwise SOCI₂ (1mL). The mixture was stirred at room temperature 1 day. The solvent was evaporated. The crude product was purified by preparative HPLC in basic conditions to yield the product as colourless oil (57 mg, 95%). Purity : 98% ; ¹H NMR (CD₃OD) δ ppm : 8.38 (s, 1H), 7.96 (s, 1H), 7.32-7.26 (m, 5H), 6.96 (s, 1H), 4.62 (m, 1H), 3.76 (d, *J* = 13.2 Hz, 1H), 3.71 (d, *J* = 13.2 Hz, 1H), 3.68 (s, 3H), 3.38 (s, 2H), 3.30 (s, 2H), 3.14 (dd, *J =* 5.9 Hz and *J =* 15.4 Hz, 1H), 3.01 (dd, J = 8.07 Hz and *J* = 15.4 Hz, 1H), 2.74 (s, 3H); ¹³C NMR (CD₃OD) δ ppm : 173.5, 173.4, 173.2, 138.8, 135.7, 133.3, 130.4, 129.6, 128.7, 118.2, 60.0, 58.6, 55.6, 53.8, 52.2, 30.2, 26.4; LC : tᵣ = 3.7min; MS (ESI+): m/z = 388 (M+H)⁺.

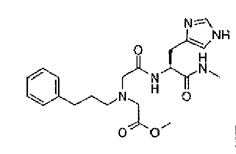

**[{[(S)-2-(1H-Imidazol-4-yl)-1-methylcarbamoyl-ethylcarbamoyl]-methyl}-(3-phenyl-propyl)-amino]-acetic acid methyl ester (91).2CF₃COOH** was prepared as followed. A solution of (4) (1 mmol) in methanol (10 mL) in trifluoroacetic anhydride 2% in acetic anhydride (5mL) was stirred 4 hours at room temperature and then evaporated. To the crude product was added 23 (1 mmol) in DMF (5mL) were added the intermediate amine (1mmol), HOBt (149mg, 1.1mmol), EDCI (211mg, 1.1mmol) and DIEA (690µL, 4mmol). The mixture was stirred overnight. Then 213mg of EDCI and 200µL of DIEA were added and the resulting slurry was stirred overnight. The solvent was evaporated. The crude product was purified by preparative HPLC to give the expected compound (171 mg, 26%). Purity: 92%; LC : tᵣ = 6.14min; MS (ESI+): m/z = 658 (M+H)⁺. To a stirred solution of triisopropylsilane and DCM (0.5mL/4mL) was added the protected compound (171 mg, 0.26 mmol). Then 500 µL of TFA were added. The mixture was stirred at room temperature for 2 hours. Solvents were evaporated and the crude product was washed with petroleum ether and diethyl ether to give the product as brown oil (165 mg, 99%). Purity : 99%; ¹H NMR (DMSO-*d6*) δ ppm : 8.94 (s, 1H), 8.51 (d, *J=* 7.9Hz, CONH), , 8.05 (q, J = 4.7Hz, CONH), 7.31-7.14 (m, 6H), 4.58 (m, 1H), 3.82 (s, 2H), 3.65 (s, 3 H), 3.62 (s, 1H), 3.13 (dd, *J =* 5.12 Hz and J = 15.3 6 Hz, 1H), 2.92 (dd, *J=* 8.4 Hz and J= 15.36Hz, 1H), 2.84 (t, J = 7.44Hz, 2H), 2.59 (d, J = 4.2Hz, 3H), 2.53 (m, 2H), 1.75 (qt, J= 7.44Hz, 2H); ¹³C NMR (DMSO-*d6*) δ ppm : 170.2, 169.2, 167.7, 141.2, 134.4, 129.4 , 128.4, 126.0, 116.9, 56.3, 54.5, 52.1, 51.5, 32.3, 27.3, 27.2, 25.3; LC : tᵣ = 4.07min; MS (ESI+) : m/z = 644 (M+H)⁺.

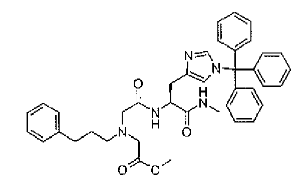

**[{[(S)-1-Methylcarbamoyl-2-(1-trityl-1H-imidazol-4-yl)-ethylcarbamoyl]-methyl}-(3-phenyl-propyl)-amino]-acetic acid methyl ester (92):** To a stirred solution of **(23)** (456mg, 1.1 mmol) in DMF (5mL) were added **(14)** (1mmol) HOBt (149mg, 1.1mmol), EDCI (211mg, 1.1mmol) and DIEA (690µL, 4mmol). The mixture was stirred overnight. Then 213mg of EDCI and 200µL of DIEA were added and the resulting slurry was stirred overnight. The solvent was evaporated. The crude product was purified by preparative HPLC to give the expected compound (171 mg, 26%).Purity : 92% ; LC : tᵣ = 6.14min; MS : (ESI+): m/z = 658 (M+H)⁺.

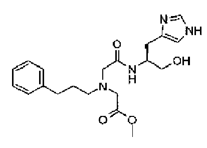

**[{[(S)-1-Hydroxymethyl-2-(1H-imidazol-4-yl)-ethylcarbamoyl]-methyl}-(3-phenyl-propyl)-amino]-acetic acid methyl ester (93) :** To a stirred solution of **L-Histidinol.2HCl** (257mg, 1.2 mmol) in DMF (5mL) were added **(14)** (1mmol), HOBT (149mg, 1.1mmol), EDCI (211mg, 1.1mmol) and DIEA (690µL, 4mmol). The mixture was stirred overnight. The solvent was evaporated. The crude product was purified by preparative HPLC to give the compound as brown oil (140 mg, 36%). Purity : 94% ; ¹H NMR (DMSO-*d6*) δ ppm : 7.68 (d, J = 8.4Hz, 1H), 7.49 (s, 1H), 7.28-7.23 (m, 5H), 6.76 (s, 1H), 3.97 (m, 1H), 3.59 (s, 3H), 3.37 (s, 2H), 3.36 (dd, J = 4.2Hz, J = 16.9Hz, 1H), 3.29 (dd, J = 5.4Hz, J = 16.9Hz, 1H), 3.12 (s, 2H), 2.75 (dd, J = 6.05Hz, J = 15Hz, 1H), 2.65 (dd, J = 7.2Hz, J = 15Hz, 1H), 1.65 (qt, J₇₋₆ = 7.7Hz, 2H); ¹³C NMR (DMSO-*d6*) δ ppm : 171.4, 169.7, 142.0, 134.6, 128.2, 125.6, 62.3, 58.0, 54.8, 53.9, 51.2, 50.1, 35.8, 32.6, 30.8; LC : tᵣ = 4.08min; MS : (ESI+) : m/z = 389 (M+H)⁺.

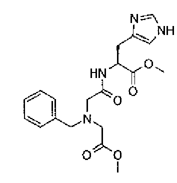

**(S)-2-[2-(Benzyl-methoxycarbonylmethyl-amino)-acetylamino]-3-(1H-imidazol-4-yl)-propionic acid methyl ester (94) : (24)** (60mg, 0.16 mmol) was stirred overnight at room temperature in a 20 % SOCl₂ solution in MeOH (2 mL) and the solvent was evaporated. The crude product was purified by preparative HPLC to yield compound as a colorless oil (50 mg, 73%). Purity 100%; ¹H NMR (MeOD) δ ppm: 7.87 (s, 1H), 7.33-7.25 (m, 5H), 6.97 (s, 1H), 7.75 (m, 1H), 3.77 (d, *J*=13.2Hz*,* 1H), 3.71 (d, *J=*13.2 Hz, 1H), 3.73 (s, 3H), 3.67 (s, 3H), 3.36 (s, 2H), 3.31 (s, 2H), 3.21 (dd, *J*=4.5 and 14.1 Hz, 1H), 3.13 (dd, *J*=7.5 and 15.0 Hz, 1H). LC t_{R}= 3.42 min, MS (ESI+): m/z= 389(M+H)⁺.

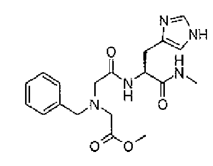

**(Benzyl-{[(S)-2-(1H-imidazol-4-yt)-1-methylcarbamoyl-ethylcarbamoyl]-methyl}-amino)-acetic acid methyl ester (95):** To a stirred solution of (79) (86mg, 0.14mmol) in methanol (4mL) at 0°C were added dropwise SOCl₂ (1mL). The mixture was stirred at room temperature 1 day. The solvent was evaporated. The crude product was purified by preparative HPLC in basic conditions to yield the product as colourless oil (57 mg, 95%). Purity : 98%; ¹H NMR (CD₃OD) δ ppm : 8.38 (s, HCOOH), 7.96 (s, 1H), 7.32-7.26 (m, 5H), 6.96 (s, 1H), 4.62 (m, 1H), 3.76 (d, J - 13.2 Hz, 1H), 3.71 (d, J = 13.2 Hz, 1H), 3.68 (s, 3H), 3.38 (s, 2H), 3.30 (s, 2H), 3.14 (dd, J = 5.9 Hz, J = 15.4 Hz, 1H), 3.01 (dd, J = 8.07 Hz, J = 15.4 Hz, 1H), 2.74 (s, 3H); ¹³C NMR (CD₃OD) δ ppm: 173.5, 173.4, 173.2, 138.8, 135.7, 133.3, 130.4, 129.6, 128.7, 118.2, 60.0, 58.6, 55.6, 53.8, 52.2, 30.2, 26.4; LC : tᵣ = 3.7min; MS : (ESI+): m/z = 388 (M+H)⁺.

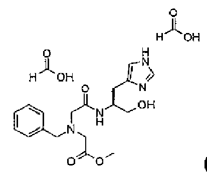

**(Benzyl-{[(S)-2-hydroxy-1-(1H-imidazol-4-ylmethyl)-ethylcarbamoyl]-methyl}-amino)-acetic acid methyl ester (96):** To a stirred solution of **L-Histidinol.2HCl** (219mg, 1 mmol) in DMF (5mL) were added **13** (260mg, 1.1mmol), HOBt 168mg, 1.1mmol), EDCI (210 mg, 1.1mmol) and TEA (306µL, 2.2mmol). The mixture was stirred 2 hours. The solvent was evaporated. The crude product was solubilised in DCM and washed with a saturated solution of NaHCO₃. The aqueous layers were extracted with DCM and AcOEt and the organic layers were dried with MgSO₄ and evaporated. The crude product was purified preparative HPLC to yield compound as colourless oil (257mg, 71%). Purity: 95% ; ¹H NMR (DMSO-*d6*) δ ppm: 8.32 (s, 2HCOOH), 7.75 (d, J = 8.65 Hz, 1H), 7.52 (d, J = 1.2 Hz, 1H), 7.31-7.25 (m, 5H), 6.76 (d, J = 1.2Hz, 1H), 3.96 (m, 1H), 3.70 (s, 2H), 3.59 (s, 3H), 3.35 (dd, J = 4.9 Hz, J = 10.5 Hz, 1H), 3.34 (s, 2H), 3.27 (dd, J = 5.5 Hz, J = 10.5 Hz, 1H), 3.17 (s, 2H), 2.75 (dd, J = 5.5 Hz, J = 14.8 Hz, 1H), 2.68 (dd, J = 6.8 Hz, J = 14.8 Hz, 1H); ¹³C NMR (DMSO-*d6*) δ ppm : 171.1, 169.2, 164.7, 138.0, 134.7, 134.1, 128.9, 128.3, 127.3, 117,6, 62.2, 57.7, 57.0, 53.8, 51.3, 50.2, 28.2; LC : tᵣ = 3.54min; MS : (ESI+): m/z = 361 (M+H)⁺.

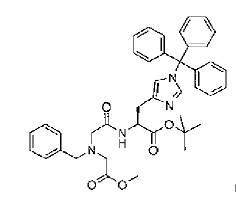

**(S)-2-[2-(Benzyl-methoxycarbonylmethyl-amino)-acetylamino]-3-(1-trityl-1H-imidazol-4-yl)-propionic acid tert-butyl ester (97):** To a stirred solution of **H-L-His(Trt)OtBu** (226mg, 0.5 mmol) in DMF (5mL) were added **13** (140mg, 0.59mmol), HOBt (92mg, 0.59mmol), EDCI (115 mg, 0.59mmol) and TEA (160µL, 1.15mmol). The mixture was stirred overnight. The solvent was evaporated. The crude product was precipitated in water. The filtrate was purified by preparative HPLC to yield the expected compound (218mg, 64%). Purity : 98% ; ¹H NMR (DMSO-*d6*) δ ppm : 8.54 (d, J = 8.1Hz, 1H), 7.38-7.01 (m, 15H+5H+1H), 6.68 (s, 1H), 4.46 (m, 1H), 3.81 (d, J = 13.2 Hz, 1H), 3.75 (d, J = 13.2 Hz, 1H), 3.55 (s, 3H), 3.33 (s, 2H), 3.25 (s, 2H), 2.92-2.87 (m, 2H), 1.28 (s, 9H); LC : tᵣ = 6.48min; MS : (ESI+) : m/z = 673 (M+H)⁺.

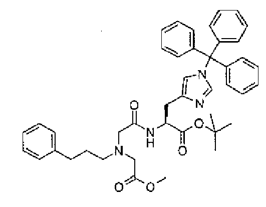

**(S)-2-{2-[Methoxycarbonylmethyl-(3-phenyl-propyl)-amino]-acetylamino}-3-(1-trityl-1H-imidazol-4-yl)-propionic acid tert-butyl ester (98):** To a stirred solution of **H-L-His(Trt)OtBu** (317mg, 0.7 mmol) in DMF (5mL) were added **14** (0.77mmol), HOBt (117mg, 0.77mmol), EDCI (148 mg, 0.77mmol) and TEA (214µL, 1.54mmol). The mixture was stirred overnight. The solvent was evaporated. The crude product was precipitated in water. The filtrate was purified by preparative HPLC to yield the expected compound (257mg, 47%). Purity : 98% ; ¹H NMR (DMSO-*d6*) δ ppm : : 8.39 (d, J = 8.4Hz, 1H), 7.36-6.99 (m, 15H+5H+1H), 6.65 (s, 1H), 4.45 (m, 1H), 3.54 (s, 3H), 3.43 (s, 2H), 3.18 (s, 2H), 2.87 (m, 2H), 2.60-2.54 (m, 2H+2H), 1.65 (qt, J = 7.6Hz, 2H), 1.29 (s, 9H); LC : tᵣ = 6.71min; MS : (ESI+) : m/z = 701 (M+H)⁺.

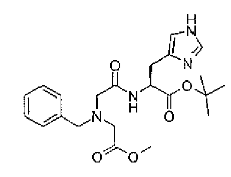

**(S)-2-[2-(Benzyl-methoxycarbonylmethyl-amino)-acetylamino]-3-(1H-imidazol-4-yl)-propionic acid tert-butyl ester (99):** To a stirred solution of TIS and DCM (1mL/8mL) was added **97** (210 mg, 0.31 mmol). Then 1mL of HCOOH were added. The mixture was stirred at room temperature overnight. The mixture was washed with water. The aqueous layer was lyophilised and the crude product was washed with petroleum ether and diethyl ether to yield the compound as colourless oil (119 mg, 89%). Purity : 98% ; ¹H NMR (DMSO-*d6*) δ ppm : 8.33 (d, J = 7.8Hz, 1H), 7.66 (s, 1H), 7.35-7.23 (m, 5H), 6.87 (s, 1H), 4.45 (m, 1H), 3.80 (d, J = 13.2 Hz, 1H), 3.74 (d, J = 13.2 Hz, 1H), 3.62 (s, 3H), 3.37 (s, 2H), 3.24 (s, 2H), 2.98 (dd, J = 6.6 Hz, J = 14.7 Hz, 1H), 2.90 (dd, J = 5.4 Hz, J = 14.7 Hz, 1H), 1.28 (s, 9H); ¹³C NMR (DMSO-*d6*) δ ppm : 171.0, 170.3, 169.7, 138.0, 135.0, 128.9, 128.3, 127.3, 122.9, 80.6, 57.6, 56.7, 53.3, 52.3, 51.2, 28.9, 27.5; LC : tᵣ = 4.77min; MS : (ESI+) : m/z = 431 (M+H)⁺.

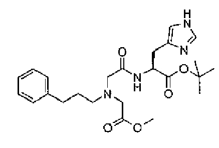

**(S)-3-(1H-Imidazol-4-yl)-2-{2-[methoxycarbonylmethyl-(3-phenylpropyl)-amino]-acetylamino}-propionic acid tert-butyl ester (100):** To a stirred solution of TIS and DCM (1mL/8mL) was added **(98)** (251 mg, 0.36 mmol). Then 1mL of HCOOH were added. The mixture was stirred at room temperature overnight. The mixture was washed with water. The aqueous layer was lyophilised and the crude product was washed with petroleum ether and diethyl ether. The product was dissolved in methanol and relarged with diethyl ether to yield the compound as colourless oil (110 mg, 67%).Purity : 98% ; ¹H NMR (DMSO-*d6*) δ ppm : 8.14 (d, J = 8.6Hz, 1H), 7.59 (s, 1H), 7.28-7.12 (m, 5H), 6.86 (s, 1H), 4.44 (m, 1H), 3.59 (dt, J = 6.3Hz, J = 8.6Hz, 1H), 3.44 (s, 2H), 3.20 (s, 2H), 2.91 (d, J = 6.3Hz, 2H), 2.61-2.52 (m, 2H+2H), 1.67 (qt, J = 7.8Hz, 2H), 1.32 (s, 9H); ¹³C NMR (DMSO-*d6*) δ ppm: 171.3, 170.2, 170.1, 142.0, 134.9, 133.0, 128.3, 128.2, 125.6, 116.5, 80.6, 57.5, 54.6, 53.8, 52.2, 51.2, 32.6, 29.3, 29.2, 27.5; LC : tᵣ = 4.95min; MS : (ESI+) : m/z = 459 (M+H)⁺.

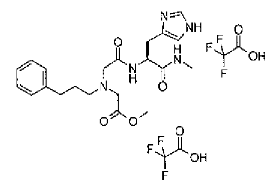

**[{[(S)-2-(1H-Imidazol-4-yl)-1-methylcarbamoyl-ethylcarbamoyl]-methyl}-(3-phenyl-propyl)-amino]-acetic acid methyl ester (101).2CF₃COOH** To a stirred solution of TIS and DCM (0.5mL/4mL) was added (65) (171 mg, 0.26 mmol). Then 0.5mL of TFA were added. The mixture was stirred at room temperature during 2 hours. Then the solvent was evaporated and the crude product was washed with petroleum ether and diethyl ether to give the product as brown oil (165 mg, 99%).Purity : 99% ; ¹H NMR (DMSO-*d6*) δ ppm: 8.94 (s, 1H), 8.51 (d, J = 7.9Hz, 1H),, 8.05 (q, J = 4.7Hz, 1H), 7.31-7.14 (m, 5H + 1H), 4.58 (m, 1H), 3.82 (s, 2H), 3.65 (s, 3H), 3.62 (s, 1H), 3.13 (dd, J = 5.12 Hz, J = 15.36 Hz, 1H), 2.92 (dd, J = 8.4 Hz, J = 15.36Hz, 1H), 2.84 (t, J = 7.44Hz, 2H), 2.59 (d, J = 4.2Hz, 3H), 2.53 (m, 2H), 1.75 (qt, J = 7.44Hz, 2H); ¹³C NMR (DMSO-*d6*) δ ppm : 170.2, 169.2, 167.7, 141.2, 134.4, 129.4, 128.4, 126.0, 116.9, 56.3, 54.5, 52.1, 51.5, 32.3, 27.3, 27.2, 25.3; LC : tᵣ = 4.07min; MS : (ESI+) : m/z = 644 (M+H)⁺.

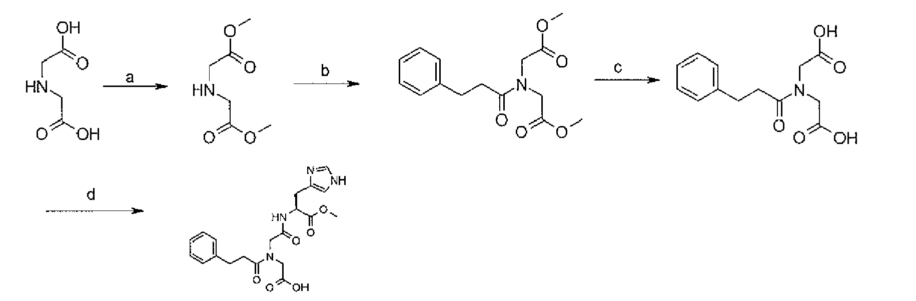

**Scheme 9 :** Reactants and conditions : a) SOCl₂, MeOH, room temperature, overnight b) hydrocinnamoyl chloride, DIEA, DCM, room temperature, overnight c) NaOH, MeOH, H₂O d) trifluoroacetic anhydride 2% in acetic anhydride, 20-70°C, 5 h, 100% then L-His-OMe, DIEA, anh.DMF, argon room temp, overnight.

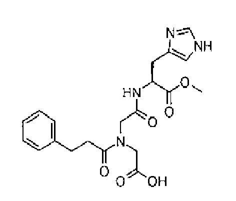

**(S)-2-{2-[Carboxymethyl-(3-phenyl-propionyl)-amino]-acetylamino}-3-(1*H*-imidazol-4-yl)-propionic acid methyl ester (102)** Iminodiacetic acid was stirred overnight in a 80/20 MeOH/SOCl₂ mixture and evaporated. To the diester obtained (591 mg, 3.0 mmol) in solution in CH₂Cl₂ (15 mL) were added DIEA (1.5 mL, 9.0 mmol) and the hydrocinnamoyl chloride (537 µL, 3.6 mmol). The mixture was stirred overnight at room temperature and washed with HCl 0.5 N solution and NaHCO₃ 10 % solution. Organic layer was dried over MgSO₄ and evaporated to give the desired disester as a colorless oil (99 %). The diester (3.0 mmol) was saponified with NaOH (15 mmol) in MeOH (10 mL) and H₂O (1 mL). MeOH was evaporated, HCI 1 N was added to the residue and the aqueous layer was extracted 3 times with AcOEt to give the [Carboxymethyl-(3-phenyl-propionyl)-amino]-acetic acid (99 %). Purity 98%; ¹H NMR (DMSO-*d6*) δ ppm: 7.23 (m, 5H), 4.18 (s, 2H), 3.98 (s, 2H), 2.79 (t, *J=* 8.1 Hz, 2H), 2.56 (t, J= 8.1Hz, 2H). LC t_{R}= 3.41 min, MS (ESI+): m/z= 266 (M+H)⁺. [Carboxymethyl-(3-phenyl-propionyl)-amino]-acetic acid was reacted with L-His-OMe to give **102** (36%).Purity : 99 %; ¹H NMR (DMSO-d6) δ ppm : 8.89 (d, J = 7.2Hz, 1H), 7.74 (s, 0.4H), 7.61 (s, 0.6H), 7.28-7.13 (m, 5H), 6.89 (s, 0.4H), 6.84 (s, 0.6H), 4.48 (m, 1H), 4.06 (s, 2H), 3.93 (s, 2H), 3.58 (s, 1.2H), 3.54 (s, 1.8H), 2.95 (m, 2H), 2.82 (m, 2H), 2.50 (m, 2H); ¹³C NMR (DMSO-d6) δ ppm: 172.7, 172.5, 171.6, 171.5, 168.9, 141.2, 134.9, 134.7, 128.3, 128.2, 125.8, 116.8, 116.5, 52.5, 52.4, 51.9, 51.8, 49.5, 33.5, 30.3, 28.5, 28.4; LC : t_{R}= 3.25 min; MS : (ESI+): m/z= 417 (M+H)⁺.

### Example 10: Material and methods for evaluating the activity and binding of a ligand of the exosite of IDE

### 1) Reagents and substrates

Assay buffer was HEPES at 50 mM with 100 mM NaCl, pH 7.4. HEPES, NaCl and EDTA were purchased from Sigma Aldrich (St Louis, MO, USA) Ac-Cys-Lys-Leu-Val-Phe-Phe-Ala-Glu-Asp-Trp-NH2 was synthesized by NeoMPS (PolyPeptide Lab., Strasbourg, France). The labelled substrate prepared in house was ATTO 655- Cys-Lys-Leu-Val-Phe-Phe-Ala-Glu-Asp-Trp where Cys was used as a linker and Trp as a quencher. Small aliquots of the lyophilized peptide, stored at -20°C, were extemporaneously solubilized to a concentration of 1.25 mM with DMSO and diluted to 10 µM in HEPES buffer. After sonication and centrifugation (5 minutes, 3000g), the supernatant was immediately used in the enzymatic assay. Small aliquots of recombinant human IDE solution (21) stored at - 80°C, were extemporaneously solubilized to a concentration of 2.5µg/mL with the HEPES buffer to be dispensed into the assay plates.

### 2) Evaluation of IDE activity by the method of identifying a modulators of IDE, using fluorogenic amyloid β 16-23 as a substrate

The enzymatic activity of IDE was assayed using a fluorogenic peptide (ATTO 655- Cys-Lys-Leu-Val-Phe-Phe-Ala-Glu-Asp-Trp) close to amyloid β 16-23 (Lys-Leu-Val-Phe-Phe-Ala-Glu-Asp) and a method related to the protocol described in EP 1 674 580 20 µL of IDE were pre-incubated 10 minutes at ambient temperature with 20 µL of test compound or vehicle in 96 well microtiter plates (dark, non-binding surface). The reaction was then started with the addition of 40 µL of substrate. Final concentrations of IDE, compounds and substrate were 0.625 µg/mL, 30 µM and 5 µM, respectively. Final DMSO was 1.4 %. EDTA 2mM was used as a reference inhibitory control. Incubations were performed for 40 minutes at 37°C. For the kinetic readout a Victor 3V (Perkin-Elmer) was used with excitation at 635 nm and emission at 750 nm.

### 3) X rays diffraction method to evaluate binding of a compound to exosite of IDE

The expression of cysteine free human IDE (IDE-CF; C110L, C171S, C178A, C257V, C414L, C573N, C590S, C789S, C812A, C819A, C904S, C966N, C974A) and the catalytically inactive IDE-CF-E111Q mutants was performed using E. coli Rosetta (DE3) cells (at 25 °C and 19 hours, IPTG induction) and purified by Ni-NTA, Source-Q, and 3-5 cycles of Superdex S-200 columns (22). IDE-CF-E111Q in complex with inhibitor or activator was crystallized by hanging drop vapor diffusion at 18 °C, using 1 µl of protein (16-20 mg/ml) and 1 µl of mother liquor (10-13% PEG MME 5000, 100 mM HEPES pH 7.0, 4-14% Tacsimate, 10% dioxane). Clusters of needle crystals appeared in 3 to 5 days and were equilibrated in cryo-protective buffer containing 30% glycerol and mother liquor and were flash frozen in liquid nitrogen. Diffraction data were collected at 100K at the Advance Photon Source 14-BM-C and 19-ID beamlines at Argonne National Laboratory. The data sets were processed using HKL2000. The structures were solved by molecular replacement (Phaser) using the IDE portion of Aβ-bound IDE-E111Q structure as a search model (PDB: 2G47). Structure refinement and rebuilding were performed using REFMAC and Coot. The extra electron density at the catalytic chamber of IDE in the structures of IDE in complex with inhibitor/activator were clearly visible based on sA-weighted Fo-Fc map calculated by CNS and manually built.

The co-crystallization was performed with compounds: **24, 25, 29, 69, 80, 102.**

### Example 11: in vitro activities of IDE on the substrate amyloid β 16-23, in the presence of the compounds of the invention.

^{a}_{:} -Log(IC₅₀ M).

| Cpd No | plC₅₀^{a} | Cpd No | plC₅₀^{a} | Cpd No | plC₅₀^{a} |
|---|---|---|---|---|---|
| **24** | 5.9 | **44** | 5.1 | **68** | 5.9 |
| **29** | 62 | **45** | 6.2 | **69** | 6.2 |
| **30** | 5.2 | **46** | 6.6 | **70** | 5.8 |
| **31** | 5.8 | **47** | 5.2 | **71** | 5.5 |
| **32** | 5.6 | **48** | 6.1 | **72** | 6.4 |
| **33** | 6.2 | **49** | 5.4 | **73** | 6.4 |
| **34** | 6.7 | **50** | 6.5 | **74** | 6.1 |
| **35** | 4.4 | **51** | 5.4 | **75** | 6.4 |
| **36** | 5.4 | **52** | 6.4 | **76** | 5.9 |
| **37** | 4.4 | **53** | 5.8 | **77** | 51 |
| **38** | 6.3 | **55** | 6.2 | **78** | 5.5 |
| **39** | 61 | **56** | 5.1 | **79** | 6.1 |
| **40** | 5.9 | **57** | 6.3 | **80** | 7.6 |
| **41** | 6.0 | **63** | 5.2 | **81** | 4.9 |
| **42** | 4.4 | **66** | 5.9 | **85** | 6.2 |
| **43** | 4.6 | **67** | 5.1 | | |

### Example 12: Kinetics of IDE substrate hydrolysis and modulation of IDE 24 and 80 using other substrates than amyloid-β.

### 1) Methods

The enzymatic activity of IDE was assayed using human insulin, IGF-2 or somatostatin, as substrates in Hepes 50 mM with 100 mM NaCl, pH 7.4.

20 µL of IDE at 30 µg/mL were pre-incubated 10 minutes at 37°C with 20 µL of vehicle or modulator (400µM) in 96-well microtiter plates (black, low-binding). The reaction was then started with the addition of 40 µL of substrate at 4 µM. Final concentrations of IDE, substrate, modulators were 7.51 µg/mL, 1 µM, 200µM respectively. The reaction was quenched at 1, 2, 10, 20 and 30 minutes with 20µL of a solution of TFA/ACN 0.1%. Quantification of residual substrate was performed by HPLC analysis (UV 215 nm) using a UltiMate® nano LC system apparatus (micropump, solvent organizer, UV detector with U-Z view capillary cell), equipped with a Famos® autosampler and a Switchos® precolumn switching device all from LC_packings (Dionex.Corp). 10µL of sample were loaded on a precolumn 5*0.3 mm equipped with a guard cartridge Q95961 10*1.0 mm from Interchim at a flowrate of 50µL/min for preconcentration. Separation was performed using an Uptisphere UPSWTF-A10 C18wtf column 5 µm particle size column, dimensions 0.3 * 100 mm from Interchim at 50°C. A gradient starting from 75% H₂O/0.1% TFA and reaching 75% CH₃CN /0.09% TFA within 10 min at a flow rate of 0.3 µL/min was used and quantification was done at 215/254nm.

### 2) Results: Activities using various substrates.

| | **80** | **24** |
|---|---|---|
| **ATTO 655- Cys-Lys-Leu-Val-Phe-Phe-Ala-Glu-Asp-Trp** | **inhibition** | **inhibition** |
| **Insulin** | **activation** | **activation** |
| **Somastatin** | **no effect** | **no effect** |
| **IGF-2** | **activation** | **activation** |

These results show that, depending on the substrate, the same product may be an activator of IDE, an inhibitor of IDE or a ligand of IDE without any detectable enzymatic activity on the selected substrate.

### Bibliography

1. Duckworth, W. C. et al., Endocr Rev 1998, 19, (5), 608-24.
2. Hersh, L., CMLS) 2006, 63, (21), 2432.
3. Kurochkin, I. V., Trends in Biochemical Sciences 2001, 26, (7), 421.
4. Malito, E. et al. CMLS, 2008, 65, (16), 2574-85.
5. Mirsky, I. A.et al. Arch Biochem 1949, 20, (1), 1-9.
6. Kurochkin, I. V. et al. FEBS Lett 1994, 345, (1), 33-7.
7. Farris, W. et al., PNAS, 2003, 100, (7), 4162-7.
8. Farris, W. et al. Am J Pathol 2004, 164, (4), 1425-34.
9. Miller, B. C. et al., PNAS, 2003, 100, (10), 6221-6.
10. Sladek, R.et al., Nature 2007, 445, (7130), 881-5.
11. Vepsalainen, S. et al., J Med Genet 2007, 44, (9), 606-8.
12. Florez, J. C. et al., Diabetes 2006, 55, (1), 128-35.
13. Bertram L, B. D. et al., Science 2000, 22, (290(5500)), 2302-3.
14. Qiu, W. Q. et al., Neurobiol Aging 2006, 27, (2), 190-8.
15. Prince, J. A.et al., Hum Mutat 2003, 22, (5), 363-71.
16. Kurochkin, I. V., FEBS Lett 1998, 427, (2), 153-6.
17. Im, H. et al., J Biol. Chem. 2007, 282, (35), 25453-25463.
18. Shen, Y. et al., Nature 2006, 443, (7113), 870-4.
19. Malito E. et al., Biochem., 2008, 47,12822-12634.
20. Li P. et al., BBRC, 2006, 343, 4, 1032-1037.
21. Shen Y. et al., Nature, 2006, 443, 870-874.
22. Manolopoulou et al, JBC, 284:14177, 2009**.**
23. Im H. et al., JBC, 2007, 282, 35, 25453-25463.
24. Cabrol C. et al., PloS ONE, 2009, 4, 4, e5274, 1-8.
25. Leissring M. et al., Biochem. J., 2004, 383, 439-446.
26. Kim M. et al., JBC, 2007,282,11, 7825-7832.
27. Poulain, R. F.et al. Tetrahedron Letters, 2001, 42(8) : 1495-1498.
28.: Clemencon, Isabelle F. et al., Tetrahedron, 2007, 63; 35; 8665 - 8669.
29. Tully, W. Roger et al., , J. Med. Chem., 1991, 34 (7), 2060-2067.
30. Padmavathi, V. et al., Eur. J. Med. Chem.; 2009, 44; 5; 2106 - 2112.
31. Yen, T.T. et al., Arch Int Pharmacodyn Ther. 1991, 310:162-74.

## Claims

1. Compounds of the following formula (I): 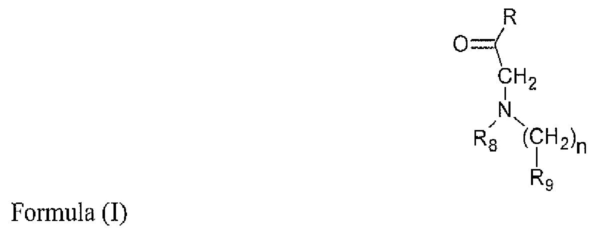 wherein:
R₈ is chosen among:
• H,
• a linear or branched C₁-C₇ alkyl group, or
• a (linear or branched C₁-C₆ alkyl)-Ar group in which Ar is chosen in the group consisting of a phenyl, naphtyl, quinolein, indole, benzimidazole, benzothiazole, pyridine, pyrimidine, pyrazine group, optionally substituted with an halogen atom such as Cl, Br, I, F, a -CF₃ group, a linear or branched C₁-C₆ alkyl group, or a -O-(C₁-C₆ alkyl) group, ,
_{.} a (linear or branched C₁-C₅ alkyl)-O-Ar group in which Ar is chosen in the group consisting of a phenyl, naphtyl, quinolein, indole, benzimidazole, benzothiazole, pyridine, pyrimidine, pyrazine group, optionally substituted with an halogen atom such as Cl, Br, I, F, a ―CF₃ group, a linear or branched C₁-C₆ alkyl group, or a ―O-(C₁-C₆ alkyl) group,
**R**₉ is chosen in the group consisting of
_{.} a -COR' group in which R' is chosen among -OH, -O-(linear or branched C₁-C₄ alkyl) or-NHOH,
_{.} an isoster of a ―COOH group, preferably a tetrazole, 1,2,4-oxadiazole-5-one group, a 1,2,4-oxadiazole-5-thione,
_{•} an hydroxamic acid group (-CONHOH),
_{•} a squaric acid group,
at the provisos that when R₉ is a squaric group, then n = 0, and when R₉ is different from a squaric acid group, then n = 1 or 2, and
**R** is a (L) or (D) aminoacid derivative having the following formula (II): 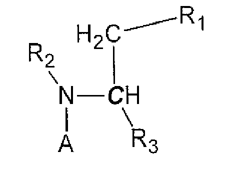 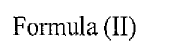 wherein:
- ***C*** designates a chiral carbon atom,
- A indicates the point of bonding of R to the corresponding carbon atom of the compound of formula I,
- **R**₁ is chosen in the group consisting of
_{•} a 4-imidazole group which is non substituted, or N-substituted, preferably in tau position, by a methyl, benzyl or trityl group,
_{.} a S-linear or branched C₁-C₇ alkyl group, preferably a phenyl group, and optionally substituted by a C₅-C₁₀ aromatic group, preferably a S-trityl group,
_{.} a ―CH₂-CH₂-guanidine group,
- **R**₂ is H or CH₃, and
- **R**₃ is chosen in the group consisting of
_{•} a -COOR₄ group in which R₄ is H or a linear or branched C₁-C₇ alkyl group, or a C₁-C₇ cycloalkyl group,
_{.} a ―CONR₅R₆ group in which R₅ and R₆ are identical or different and are independently chosen in the group consisting of H, a C₁-C₇ alkyl group, a C₁-C₇ cycloalkyl group, and a benzyl group,
_{.} a ―CH₂OH group, or
_{•} a group of the following formula: 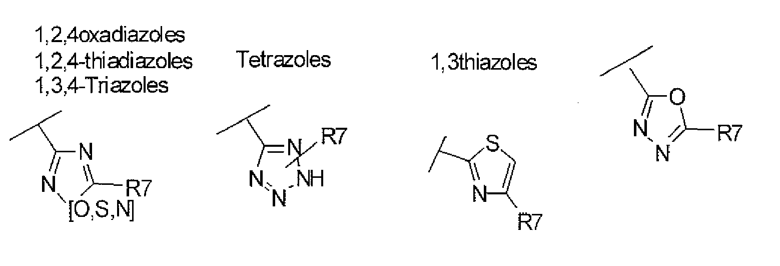
in which R₇ is a linear or branched C₁-C₇ alkyl group, and their pharmaceutically acceptable salts.

2. Compounds according to claim 1 wherein R₉ is -COR', with R' as defined in claim 1.

3. Compounds according to claim 2 wherein R₈ is a (linear or branched C₁-C₆alkyl)-Ar group, as defined in claim 1.

4. Compounds according to claim 2 wherein R₁ is a 4-imidazole group which is not substituted or N-substituted, preferably in tau position, by a methyl, benzyl or trityl group.

5. Compounds according to claim 3 or 4 wherein R₃ is a ―CONR₅R₆ group in which R₅ and R₆ are identical or different and are independently chosen in the group consisting of H, a C₁-C₇ alkyl group, a C₁-C₇ cycloalkyl group, and a benzyl group.

6. Compounds according to anyone of the preceding claims, wherein R₂ is H.

7. Compounds according to anyone of the preceding claims, having the following formula 1-6: 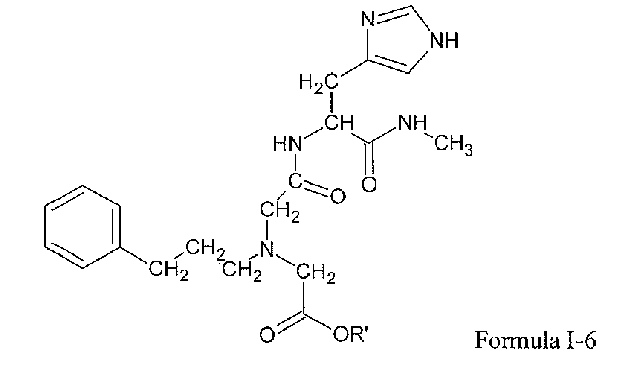 in which R' is H or methyl.

8. Method for screening molecules susceptible to modulate the activity of IDE comprising:
(i) contacting IDE with a labelled reference compound of Formula I, having a pIC₅₀ >6 and preferably >7 and a compound to be tested and
(ii) measuring the change in strength of the signal emitted by the label, a decrease in the strength of said signal being correlated to the binding of said compound to be tested to the exosite of IDE.

9. Method according to claim 8, **characterized in that** the reference compound is compound No80.

10. Method according to claim 8 or 9, **characterized in that** the labelling is selected in the group consisting of a radioactive molecule or a fluorophore.

11. A pharmaceutical composition comprising a compound of Formula I according to anyone of claims 1-7 and at least one pharmaceutical acceptable vehicle.
